(19) Europäisches Patentamt
European Patent Office
Office européen des brevets

(11) EP 4 130 004 A1

(12) EUROPEAN PATENT APPLICATION
published in accordance with Art. 153(4) EPC

(43) Date of publication:
**08.02.2023 Bulletin 2023/06**

(21) Application number: **21777116.1**

(22) Date of filing: **26.03.2021**

(51) International Patent Classification (IPC):
***C07D 487/14*** *(1974.07)* ***A61K 31/4375*** *(2000.01)*
***A61K 31/437*** *(2000.01)* ***A61P 35/00*** *(2000.01)*

(52) Cooperative Patent Classification (CPC):
**A61K 31/437; A61K 31/4375; A61P 35/00;**
**C07D 487/14**

(86) International application number:
**PCT/CN2021/083204**

(87) International publication number:
**WO 2021/190623 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.03.2020 CN 202010234026**
**22.03.2021 CN 202110304723**

(71) Applicant: **Betta Pharmaceuticals Co., Ltd**
**Yuhang**
**Hangzhou**
**Zhejiang 311100 (CN)**

(72) Inventors:
- **GAO, Jinheng**
  **Beijing 100176 (CN)**
- **XU, Xiaofeng**
  **Beijing 100176 (CN)**
- **CHEN, Liang**
  **Hangzhou, Zhejiang 311100 (CN)**
- **SUN, Zhongxin**
  **Beijing 100176 (CN)**
- **ZHANG, Yun**
  **Beijing 100176 (CN)**
- **LIU, Xiangyong**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **HGF**
**HGF Limited**
**1 City Walk**
**Leeds LS11 9DX (GB)**

# (54) SALT AND CRYSTALLINE FORMS OF FGFR4 INHIBITOR AND USES THEREOF

(57) The present invention relates to a crystalline form and/or maleate, phosphate, L-tartrate, and adipate of the compound represented by the structural formula I, and various crystalline forms of various salt forms, and preparation methods and applications thereof.

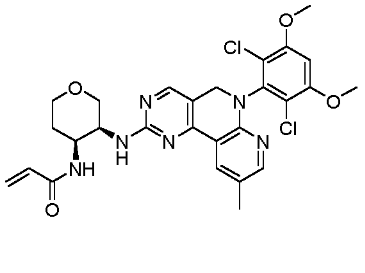

Formula I

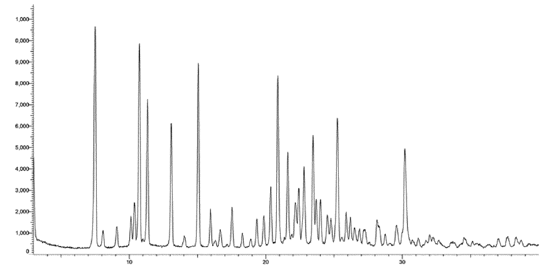

FIG. 1

EP 4 130 004 A1

## Description

TECHNICAL FIELD

The present disclosure relates to a novel crystal form, a salt form and a crystal form of the salt form of *N*-((3*S*,4*S*)-3-((6-(2,6-dichloro-3,5-dimethoxyphenyl)-9-methyl-5,6-dihydropyrimido[5,4-*c*][1,8] naphthyridin-2-yl)amino)tetrahydro-2*H*-pyran-4-yl)acrylamide. The present disclosure further relates to a pharmaceutical composition comprising the crystal form or the salt form, a use of the salt form, crystal form and pharmaceutical composition as an FGFR4 inhibitor and a method for treating an FGFR4-mediated disease.

BACKGROUND

Fibroblast growth factor receptors (FGFRs) are a member of the receptor tyrosine kinase (RTK) superfamily. FGFRs bind to fibroblast growth factors (FGFs) to regulate cell proliferation, differentiation and migration through complex signaling pathways in different tissues (Jouanneau J et al. Oncogene, 1999, 18: 327-333). Each FGFR is a single-chain glycoprotein consisting of an extracellular region, a single transmembrane region and an intracytoplasmic tyrosine kinase region, wherein the extracellular region is composed of a leader peptide and three immunoglobulin domains. The FGFR tyrosine kinase family includes FGFR1, FGFR2, FGFR3 and FGFR4. An FGFR4 gene is located at 5q35.1 of chromosome, which is about 11.3 kb in length with 18 exons (Kostrzewa M. Mammalian genome, 1998, 9 (2): 131-135). The FGFR4 protein is an important member of the FGFR tyrosine kinase family, and its 388th amino acid is located in the highly conserved transmembrane region of the RTK structure. The changes in the pathophysiological function of the FGFR4 protein caused by the changes of this structure can enhance the activity of tyrosine kinase. The FGFR4 protein is a type of transmembrane tyrosine kinase receptor with autophosphorylation activity, which plays an important role in the processes such as embryonic development, tissue repair and angiogenesis (Eswarakumar V P et al. Cytokine Growth Factor Rev, 2005, 16 (2): 139-149).

FGFR4 signaling pathway: Mediated by heparin or heparinoid, a ligand binds to FGFR4, causing an FGFR4 monomer to dimerize; with the tyrosine phosphorylation of the C-terminus of the cytoplasm, a kinase insertion region and a proximal membrane region, FGFR4 is activated by the phosphorylation of the kinase region of an activation loop (A loop) (Schlessinger J et al. Mol Cell, 2000, 6: 743-750). The activated FGFR4 mainly has two intracellular agents which are phospholipase C (PLC) and FGF receptor substrate 2 (FRS2) (Dailey L et al. Cytokine Growth Factor Rev, 2005, 16: 233-247).

After FGFR4 is activated, the Src homology region 2 (SH2) domain of phospholipase C binds to its activated C-terminal tyrosine so that the PLC is phosphorylated and binds to a C-terminal tyrosine site. The activated PLC hydrolyzes its substrate 4,5-diphosphate phosphatidylinositol ($PIP_2$) to form diacylglycerol (DAG) and inositol tri-phosphate ($IP_3$). $IP_3$ binds to intracellular specific receptors to stimulate an intracellular calcium pool to release $Ca^{2+}$, and $Ca^{2+}$ binds to calmodulin to activate $Ca^{2+}$/calmodulin-dependent protein kinase. Additionally, both $Ca^{2+}$ and diacylglycerol can activate members of the protein kinase C family. In addition to activating transcription factors, the secondary signal generated by $PIP_2$ hydrolysis can also activate a variety of intracellular reactions.

SOS protein binds to the Src homology region 3 (SH3) domain of growth factor receptor bound 2 (Grb2) to form a Grb2/SOS complex which can be connected to FGFR4 or bind to FGF receptor substrate 2α (FRS2α), where FRS2α is connected to a phosphotyrosine binding (PTB) domain, promoting the exchange of guanosine on Ras to make Ras into Ras-GTP to start a downstream MAPK signaling pathway.

The autophosphorylation of FGFR4 activates JAK family factors (JAK), the activated JAK cause the phosphorylation of a specific signal protein adsorption site on FGFR4, and this site can be the docking sites of signal transducer and activator of transcription (STAT) and other signaling molecules. The C-terminal tyrosine residue of an STAT protein is phosphorylated by JAK when the STAT protein is absorbed by the FGFR4 docking site, and the phosphorylated STAT protein is separated from the receptor to form a stable homodimer or heterodimer and then transferred to a nucleus to interact with gamma interferon activation site (GAS) enhancer family members to activate the transcription of target genes.

Micromolecular FGFR4 inhibitors inhibit an FGFR4-mediated proliferation signal by blocking the combination of extracellular ligand molecules with receptors or the transmission of intracellular kinase signals. Currently, there are many types of FGFR4 inhibitor under development. The FGFR4 selective inhibitor AZ709 developed by AstraZeneca shows a good inhibitory effect on cells expressing FGF19 or FGFR4 at a high level in *in vitro* experiments, but there is no obvious effect in *in vivo* experiments. The FGFR4 selective inhibitor FGF401 developed by Novartis can target FGFR4 specifically to treat the malignant tumors such as liver cancer caused by the overexpression of FGFR4. The FGFR4-specific inhibitor H3B6527 developed by H3 Biomedicine has a strong anti-tumor activity on FGF19 gene-amplified cells, and no bile acid-related adverse reactions have been found in the mouse and monkey animal models. Blueprint Medicine has developed and reported an FGFR4-specific inhibitor BLU554 to treat liver cancer and cholangiocarcinoma with the overexpression of FGFR4.

With the in-depth study of researchers on the structure-function relationship of FGFR4 and the interaction with other genes, the FGFR4 inhibitors with good specificity, a good therapeutic effect and low adverse reactions will be developed, and the use of FGFR4 molecular targeted therapy for tumors will be very meaningful.

The properties of active ingredients, such as chemical stability, solid-state stability and a storage cycle, are all very important factors for medicaments. Therefore, the exploration of new salt forms, new crystal forms or polymorphs of pharmaceutically acceptable compounds provides an opportunity to improve the overall properties of pharmaceutical products and expands the types of materials available for the design of formulation scientists. The salt forms and/or polymorphs of particular organic drug compounds have distinct physical properties such as morphology, melting point, solubility, hygroscopicity and stability due to their unique structures and thus exhibit different behaviors in pharmaceutical formulation, therapeutic activity and chemical and physical stability. Although skilled persons are motivated to explore the salt forms and/or crystal forms of particular organic drug compounds, it is to be noted that it is generally impossible to predict which salt formation or crystallization behavior the compounds will have only from their molecular structures, to predict how many crystal forms and what crystal forms are present for the particular organic drug compounds, and even to predict the structures and properties of the crystal forms.

SUMMARY

The present disclosure relates to a salt form of *N*-((3*S*,4*S*)-3-((6-(2,6-dichloro-3,5-dimethoxyphenyl)-9-methyl-5,6-dihydropyrimido[5,4-*c*][1,8 ]naphthyridin-2-yl)amino)tetrahydro-2*H*-pyran-4-yl)acrylamide, and crystal form thereof, the compound represented by Formula I.

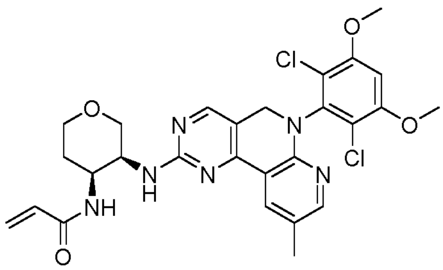

Formula I

The present disclosure provides a crystal form A of a compound represented by Formula I and a maleate, phosphate, adipate and L-tartrate of the compound represented by Formula I.

Preferably, preferred salts of the compound represented by Formula I are a maleate and a phosphate.

In another aspect, the present disclosure provides crystal forms of a maleate, L-tartrate, adipate and phosphate of a compound represented by Formula I.

In some embodiments, the present disclosure provides a crystal form A of the compound represented by Formula I, wherein an X-ray powder diffraction pattern of the crystal form A has characteristic peaks at diffraction angles 2θ of 7.5°±0.2°, 10.7°±0.2°, 11.3°±0.2° and 15.1°±0.2°.

Preferably, an X-ray powder diffraction pattern of the crystal form A has characteristic peaks at diffraction angles 2θ of 7.5°±0.2°, 10.7°±0.2°, 11.3°±0.2°, 13.1°±0.2°, 15.1°±0.2°, 20.9°±0.2° and 25.2°±0.2°.

Preferably, an X-ray powder diffraction pattern of the crystal form A has characteristic peaks at diffraction angles 2θ of 7.5°±0.2°, 10.7°±0.2°, 11.3°±0.2°, 13.1°±0.2°, 15.1°±0.2°, 20.9°±0.2°, 21.6°±0.2°, 22.8±0.2°, 23.5°±0.2°, 25.2°±0.2° and 30.2±0.2°.

Preferably, the crystal form A has an X-ray powder diffraction pattern roughly as shown in FIG. 1.

In another aspect, the crystal form A also has a thermogravimetric analysis (TGA) pattern substantially as shown in FIG. 2 and a differential scanning calorimetry (DSC) pattern substantially as shown in FIG. 3.

In some embodiments, the present disclosure provides a maleate crystal form 1 of the compound represented by Formula I, wherein an X-ray powder diffraction pattern of the maleate crystal form 1 has characteristic peaks at diffraction angles 2θ of 3.7°±0.2°, 9.9°±0.2°, 11.0°±0.2° and 16.2°±0.2°.

Preferably, an X-ray powder diffraction pattern of the maleate crystal form 1 has characteristic peaks at diffraction angles 2θ of 3.7°±0.2°, 9.9°±0.2°, 11.0°±0.2°, 13.4°±0.2°, 16.2±0.2°, 17.2°±0.2° and 20.6°±0.2°.

Preferably, an X-ray powder diffraction pattern of the maleate crystal form 1 has characteristic peaks at diffraction angles 2θ of 3.7°±0.2°, 9.9°±0.2°, 11.0°±0.2°, 13.4°±0.2°, 16.2°±0.2°, 17.2°±0.2°, 18.7°±0.2°, 19.4°±9.2°, 20.6°±0.2°, 22.6°±0.2° and 24.4°±0.2°.

Preferably, the maleate crystal form 1 has an X-ray powder diffraction pattern roughly as shown in FIG. 4.

In another aspect, the maleate crystal form 1 also has a thermogravimetric analysis (TGA) pattern substantially as shown in FIG. 5 and a differential scanning calorimetry (DSC) pattern substantially as shown in FIG. 6.

In some embodiments, a stoichiometric ratio of the maleate to the compound represented by Formula I is 1:1.

In some embodiments, the present disclosure provides a maleate crystal form 2 of the compound represented by Formula I, wherein an X-ray powder diffraction pattern of the maleate crystal form 2 has characteristic peaks at diffraction angles 2θ of 5.6°±0.2°, 7.0°±0.2° and 19.9°±0.2°.

Preferably, an X-ray powder diffraction pattern of the maleate crystal form 2 has characteristic peaks at diffraction angles 2θ of 5.6°±0.2°, 7.0°±0.2°, 9.9°±0.2°, 14.5°±0.2° and 19.9°±0.2°.

Preferably, the maleate crystal form 2 has an X-ray powder diffraction pattern roughly as shown in FIG. 7.

Preferably, the maleate crystal form 2 has a differential scanning calorimetry (DSC) pattern substantially as shown in FIG. 8.

In some embodiments, the present disclosure provides a phosphate of the compound represented by Formula I, wherein a stoichiometric ratio of the phosphate to the compound represented by Formula I is 1.5:1, which is represented by Formula II:

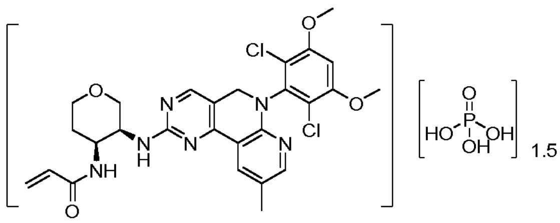

Formula II.

The present disclosure provides a crystal form 1 of Formula II, which is referred to as a phosphate crystal form 1 for convenience, and an X-ray powder diffraction pattern of the phosphate crystal form 1 has characteristic peaks at diffraction angles 2θ of 4.9°±0.2°, 10.7°±0.2°, 16.8°±0.2° and 21.3°±0.2°.

Preferably, an X-ray powder diffraction pattern of the phosphate crystal form 1 has characteristic peaks at diffraction angles 2θ of 4.9°±0.2°, 8.3°±0.2°, 10.7°±0.2°, 11.6°±0.2°, 12.8±0.2°, 16.8°±0.2° and 21.3°±0.2°.

Preferably, an X-ray powder diffraction pattern of the phosphate crystal form 1 has characteristic peaks at diffraction angles 2θ of 4.9°±0.2°, 8.3°±0.2°, 10.7°±0.2°, 11.6°±0.2°, 12.8±0.2°, 16.8°±0.2°, 20.1°±0.2°, 21.3°±0.2° and 28.5°±0.2°.

Preferably, an X-ray powder diffraction pattern of the phosphate crystal form 1 has characteristic peaks at diffraction angles 2θ of 4.9°±0.2°, 8.3°±0.2°, 10.7°±0.2°, 11.6°±0.2°, 12.8±0.2°, 16.8°±0.2°, 20.1°±0.2°, 21.3°±0.2°, 24.7°±0.2° and 28.5°±0.2°.

Preferably, the phosphate crystal form 1 has an X-ray powder diffraction pattern roughly as shown in FIG. 9 or FIG. 10.

In another aspect, the phosphate crystal form 1 also has a TGA pattern substantially as shown in FIG. 11 and a DSC pattern substantially as shown in FIG. 12.

In some embodiments, the present disclosure provides an L-tartrate crystal form 1 of the compound represented by Formula I, wherein an X-ray powder diffraction pattern of the L-tartrate crystal form 1 has characteristic peaks at diffraction angles 2θ of 5.4°±0.2°, 17.6°±0.2°, 19.7°±0.2° and 20.7°±0.2°.

Preferably, an X-ray powder diffraction pattern of the L-tartrate crystal form 1 has characteristic peaks at diffraction angles 2θ of 5.4°±0.2°, 12.9°±0.2°, 16.5°±0.2°, 17.6°±0.2°, 19.7±0.2° and 20.7°±0.2°.

Preferably, an X-ray powder diffraction pattern of the L-tartrate crystal form 1 has characteristic peaks at diffraction angles 2θ of 5.4°±0.2°, 12.9°±0.2°, 16.5°±0.2°, 17.6°±0.2°, 19.7±0.2°, 20.7°±0.2°, 22.2°±0.2° and 26.4°±0.2°.

Preferably, the L-tartrate crystal form 1 has an X-ray powder diffraction pattern roughly as shown in FIG. 13.

Preferably, the L-tartrate crystal form 1 has a TGA pattern roughly as shown in FIG. 14.

Preferably, the L-tartrate crystal form 1 has a DSC pattern roughly as shown in FIG. 15.

In some embodiments, the present disclosure provides an adipate crystal form 1 of the compound represented by Formula I, wherein an X-ray powder diffraction pattern of the adipate crystal form 1 has characteristic peaks at diffraction angles 2θ of 5.8°±0.2°, 8.4°±0.2°, 12.3°±0.2° and 22.9°±0.2°.

Preferably, an X-ray powder diffraction pattern of the adipate crystal form 1 has characteristic peaks at diffraction

angles 2θ of 5.8°±0.2°, 8.4°±0.2°, 10.0°±0.2°, 10.4°±0.2°, 12.3±0.2°, 17.5°±0.2° and 22.9°±0.2°.

Preferably, an X-ray powder diffraction pattern of the adipate crystal form 1 has characteristic peaks at diffraction angles 2θ of 5.8°±0.2°, 8.4°±0.2°, 10.0°±0.2°, 10.4°±0.2°, 12.3±0.2°, 17.5°±0.2°, 22.9°±0.2°, 25.4°±0.2° and 25.9°±0.2°.

Preferably, the adipate crystal form 1 has an X-ray powder diffraction pattern roughly as shown in FIG. 16.

Preferably, the adipate crystal form 1 has a TGA pattern roughly as shown in FIG. 17 and a DSC pattern roughly as shown in FIG. 18.

The amorphous form of the compound represented by Formula I has relatively low solubility, relatively low stability and relatively high hygroscopicity. In the present disclosure, it is found through studies that the crystal form A and the phosphate crystal form 1 of the compound represented by Formula I have relatively high stability and lower hygroscopicity; the adipate crystal form 1 and the maleate crystal form 1 have better stability. Therefore, these salts and crystal forms are beneficial for the production of medicaments and their chemical stability and safety. Additionally, it is most surprising that the phosphate crystal form 1 is prepared by a stable process and with a low cost and has the properties of low hygroscopicity, high stability and better pharmacokinetic.

Unless otherwise specified, the X-ray powder diffraction pattern is measured using the Kα line of Cu target.

Unless otherwise specified, all experiments in the present disclosure are conducted at room temperature.

The present disclosure further provides a method for preparing a compound represented by Formula I and a crystal form A, a maleate crystal form 1, a maleate crystal form 2, a phosphate crystal form 1, an adipate crystal form 1 and an L-tartrate crystal form 1 thereof.

The compound represented by Formula I may be prepared by the following route:

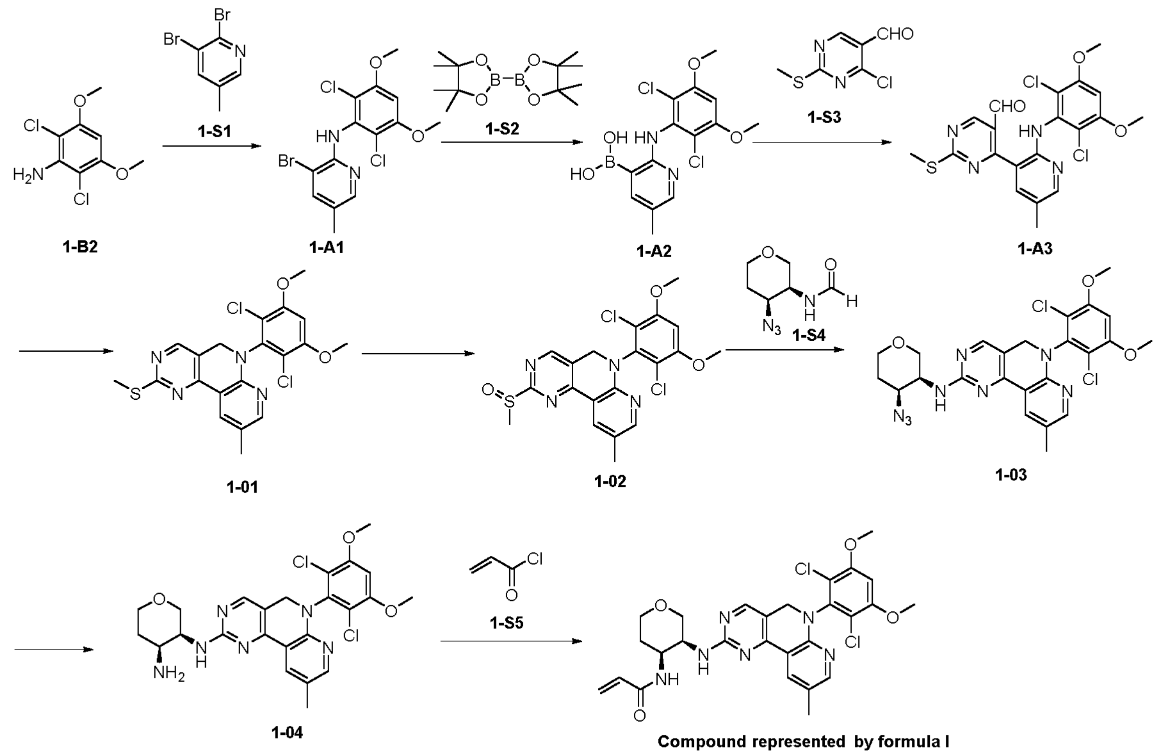

1-B2 1-A1 1-A2 1-A3
1-01 1-02 1-03
1-04
Compound represented by formula I

The specific reaction conditions of the preparation route of the compound represented by Formula I are described in specific examples. It is to be understood that the reaction solvents and conditions used in the preparation route are not limited to those used in the specific examples and other reaction conditions may also be applied to the preparation of the compound represented by Formula I.

The present disclosure further provides a pharmaceutical composition comprising a therapeutically effective amount of the salt form or crystal form of the present disclosure and a pharmaceutically acceptable excipient, adjuvant or carrier.

Secondly, the present disclosure further provides preferred embodiments of the pharmaceutical composition.

Preferably, the pharmaceutical composition contains a therapeutically effective amount of the salt form or crystal form of the present disclosure in combination with at least one other active ingredient.

Preferably, the pharmaceutical composition is used for oral administration.

Preferably, the pharmaceutical composition is used for tablets or capsules.

Preferably, the pharmaceutical composition contains 0.01 wt% to 99 wt% of the crystal form of the present disclosure.

Preferably, the pharmaceutical composition contains 10 wt% to 70 wt% of the crystal form of the present disclosure.

Preferably, the pharmaceutical composition contains 20 wt% to 50 wt% of the crystal form of the present disclosure.

The present disclosure further provides a use of the crystal form or the pharmaceutical composition for preparing a medicament.

The present disclosure further provides preferred technical solutions of the use.

Preferably, the use is treatment, prophylaxis, and delaying or preventing the occurrence or progression of cancer or cancer metastasis.

Preferably, the use is preparation of a medicament for treating an FGFR4-mediated disease.

Preferably, the disease is cancer.

Preferably, the cancer is selected from breast cancer, multiple myeloma, bladder cancer, endometrial cancer, gastric cancer, cervical cancer, rhabdomyosarcoma, non-small-cell lung carcinoma, small-cell lung carcinoma, pleomorphic lung cancer, ovarian cancer, esophageal carcinoma, melanoma, colorectal carcinoma, hepatocellular carcinoma, head and neck neoplasm, intracranial tumor, hepatocholangiocarcinoma, myelodysplastic syndrome, glioblastoma, prostate cancer, thyroid cancer, Schwann cell tumor, squamous-cell lung carcinoma, lichenoid keratosis, synoviosarcoma, skin cancer, pancreatic cancer, testicular cancer or liposarcoma.

Preferably, the use is to be used as an FGFR4 inhibitor.

The present disclosure further provides a method for treatment and/or prophylaxis of an FGFR4-mediated disease by administering a therapeutically effective amount of at least any crystal form or a pharmaceutical composition thereof to a subject in need.

Preferably, in the method, the FGFR4-mediated disease is cancer.

Preferably, in the method, the cancer is selected from breast cancer, multiple myeloma, bladder cancer, endometrial cancer, gastric cancer, cervical cancer, rhabdomyosarcoma, non-small-cell lung carcinoma, small-cell lung carcinoma, pleomorphic lung cancer, ovarian cancer, esophageal carcinoma, melanoma, colorectal carcinoma, hepatocellular carcinoma, head and neck neoplasm, intracranial tumor, hepatocholangiocarcinoma, myelodysplastic syndrome, glioblastoma, prostate cancer, thyroid cancer, Schwann cell tumor, squamous-cell lung carcinoma, lichenoid keratosis, synoviosarcoma, skin cancer, pancreatic cancer, testicular cancer or liposarcoma.

The present disclosure further provides a method for treating cancer, which comprises administering a therapeutically effective amount of at least any crystal form or a pharmaceutical composition thereof to a subject in need, wherein the cancer is breast cancer, multiple myeloma, bladder cancer, endometrial cancer, gastric cancer, cervical cancer, rhabdomyosarcoma, non-small-cell lung carcinoma, small-cell lung carcinoma, pleomorphic lung cancer, ovarian cancer, esophageal carcinoma, melanoma, colorectal carcinoma, hepatocellular carcinoma, head and neck neoplasm, intracranial tumor, hepatocholangiocarcinoma, myelodysplastic syndrome, glioblastoma, prostate cancer, thyroid cancer, Schwann cell tumor, squamous-cell lung carcinoma, lichenoid keratosis, synoviosarcoma, skin cancer, pancreatic cancer, testicular cancer or liposarcoma.

Preferably, in the method, the subject in need is human.

All crystal forms of the present disclosure are substantially pure.

In some embodiments, an acid and the compound represented by Formula I form the corresponding salts, and these salt compounds may exist in various physical forms. For example, they may exist in the form of a solution, suspension or solid. In some embodiments, a salt compound is in a solid form. The compound in a solid form may be amorphous, crystalline, co-crystalline or a mixture thereof.

In the present disclosure, the term "salt" or "salt form" may be defined as an amorphous or crystalline material where a free base API and an acid are ionized or a homogeneous crystalline (co-crystalline) material formed by the binding of the two components, a free base API and an acid, through significant intermolecular interaction such as a hydrogen bond. It is to be understood that the salts of the present disclosure may be partially ionized and partially co-crystalline.

The term "substantially pure" used herein means that the content of the crystal form is not less than 85%, preferably not less than 95%, more preferably not less than 98% by weight.

In the present disclosure, the terms "roughly" and "substantially" as used in the expression of "having an X-ray powder diffraction pattern roughly as shown in FIG. 1" or "having an X-ray powder diffraction pattern substantially as shown in FIG. 1" mean that the exact position of a peak in the figures is not to be interpreted as an absolute value. As appreciated by those skilled in the art, 2θ values in the X-ray powder diffraction pattern may have errors due to different measurement conditions (such as the equipment and instruments used) and different samples (such as samples in different batches), the measurement error of a diffraction angle in the X-ray powder diffraction pattern is 5% or lower, and generally, the difference of a given value by ±0.2° is considered appropriate. It is to be further understood that the

relative intensity of a peak may fluctuate with experimental conditions and sample preparation such as the preferred orientations of particles in a sample. The use of automatic or fixed divergence slits will also affect the calculation of the relative intensity. The intensity shown by an XRD curve included herein is exemplary only and cannot be used for absolute comparison.

It is to be understood by those skilled in the art that the data measured by DSC may vary slightly due to changes in sample purity, sample preparation and measurement conditions (such as a heating rate). It is to be understood that an alternative reading of a melting point may be given by other types of instrument or by using conditions different from those described hereinafter. Thus, the endothermic maps referenced herein are not used as absolute values, and such measurement errors are to be taken into account when DSC data is interpreted.

The term "therapeutically effective amount" used herein refers to an amount of a compound sufficient to affect the treatment of a disease, condition or symptom when the compound is administered to a subject in need for treatment of a disease or at least one clinical symptom of a disease or condition. The "therapeutically effective amount" may vary with a compound, a disease, condition and/or symptom of a disease or condition, the severity of the disease, condition and/or symptom of the disease or condition, an age of a patient treated, and/or a weight of the patient treated. In any particular case, an appropriate amount may be apparent to those skilled in the art or may be determined by conventional experiments. In the case of combined therapy, the "therapeutically effective amount" refers to a total amount of combined subjects for effectively treating a disease, condition or symptom.

The salt form or crystal form of the present disclosure may be combined as an active ingredient or mixed with a pharmaceutical carrier into a pharmaceutical composition. The pharmaceutical carrier may be in various forms, depending on the mode of administration intended, for example, oral administration or injection (including intravenous injection). Thus, the pharmaceutical composition of the present disclosure may be in the form of an individual unit suitable for oral administration, for example, capsules, wafer capsules or tablets containing a predetermined dosage of active ingredient. Further, the pharmaceutical composition of the present disclosure may be in the form of powder, granules, solutions, aqueous suspensions, non-aqueous liquids, oil-in-water emulsions or water-in-oil emulsions. Additionally, in addition to the above common dosage forms, the salt form or crystal form of the present disclosure may be administered by a controlled release and/or a delivery device. The pharmaceutical composition of the present disclosure may be prepared by any pharmaceutically acceptable method. Generally, such method includes the step of associating an active ingredient with a carrier constituting one or more essential ingredients. Generally, the pharmaceutical composition is prepared by uniformly and closely mixing an active ingredient with a liquid carrier or a finely divided solid carrier or a mixture thereof. Additionally, the product may be conveniently prepared for desired appearance.

The "pharmaceutically acceptable carrier" refers to a conventional pharmaceutical carrier suitable for a desired pharmaceutical formulation, for example, diluents and excipients such as water and various organic solvents; fillers such as starch, amylum pregelatinisatum, sucrose, dextrin, mannitol, lactose, spray-dried lactose, microcrystalline cellulose, silicified microcrystalline cellulose, and inorganic salts; binders such as starch syrup, dextrin, icing sugar, syrup, mucilage, polyethylene glycol, cellulose derivatives, alginates, gelatin, hydroxypropylcellulose, copovidone and polyvinylpyrrolidone (PVP); wetting agents such as distilled water, ethanol and glycerol; disintegrants such as dry starch, low-substituted hydroxypropyl cellulose, hydroxypropyl starch, agar, calcium carbonate, sodium bicarbonate, crospovidone, croscarmellose sodium and sodium carboxymethyl starch; absorption promoters such as quaternary ammonium compounds, amino acid ethylamine derivatives, acetoacetates, β-dicarboxylates, aromatic acid compounds and aliphatic acid compounds; surfactants such as sodium cetyl sulfate, sodium octadecyl sulfate, sodium dioctyl succinate sulfonate, sodium dodecyl sulfonate, benzalkonium bromide, benzalkonium chloride, dumefen, lecithin, cetyl alcohol, sodium dodecyl sulfate, Tween and Span; drug-loading substrates such as polyethylene glycol, carbomer, cellulose derivatives, glycogelatin, polyethylene alcohol, cocoa butter, synthetic or fully synthetic fatty acid glycerides, polyvinyl alcohol 40 stearate, petrolatum, solid paraffin, liquid paraffin, dimethicone, lanolin, beeswax and porpoise esters; absorption carriers such as kaolin and bentonite; lubricants such as talc, micronized silica gel, silicon dioxide, hydrogenated vegetable oils, magnesium dodecyl sulfate, sodium dodecyl sulfate, stearic acid, calcium stearate, magnesium stearate, sodium stearyl fumarate and polyethylene glycol. Additionally, other pharmaceutically acceptable excipients may also be added to the pharmaceutical composition, such as an anti-oxidant, a colorant, a preservative, a pH adjusting agent, a hardener, an emulsifier, a propellant, a dispersing agent, a stabilizer, a thickener, a complexing agent, a buffer, an osmotic promoter, a polymer, a fragrance, a sweetener, and a dye. Excipients suitable for the desired dosage form and the desired mode of administration are preferably used.

The term "disease" or "disorder" or "symptom" refers to any disease, disorder, condition, symptom or indication.

## BRIEF DESCRIPTION OF DRAWINGS

FIG. 1 is an X-ray powder diffraction pattern of a crystal form A of a compound represented by Formula I.

FIG. 2 is a thermogravimetric analysis (TGA) pattern of a crystal form A of a compound represented by Formula I.

FIG. 3 is a differential scanning calorimetry (DSC) pattern of a crystal form A of a compound represented by Formula I.

FIG. 4 is an X-ray powder diffraction pattern of a maleate crystal form 1 of a compound represented by Formula I.

FIG. 5 is a thermogravimetric analysis (TGA) pattern of a maleate crystal form 1 of a compound represented by Formula I.

FIG. 6 is a differential scanning calorimetry (DSC) pattern of a maleate crystal form 1 of a compound represented by Formula I.

FIG. 7 is an X-ray powder diffraction pattern of a maleate crystal form 2 of a compound represented by Formula I.

FIG. 8 is a DSC pattern of a maleate crystal form 2 of a compound represented by Formula I.

FIG. 9 is an X-ray powder diffraction pattern of a phosphate crystal form 1 of a compound represented by Formula I.

FIG. 10 is an X-ray powder diffraction pattern of a phosphate crystal form 1 of a compound represented by Formula I.

FIG. 11 is a TGA pattern of a phosphate crystal form 1 of a compound represented by Formula I.

FIG. 12 is a DSC pattern of a phosphate crystal form 1 of a compound represented by Formula I.

FIG. 13 is an X-ray powder diffraction pattern of an L-tartrate crystal form 1 of a compound represented by Formula I.

FIG. 14 is a TGA pattern of an L-tartrate crystal form 1 of a compound represented by Formula I.

FIG. 15 is a DSC pattern of an L-tartrate crystal form 1 of a compound represented by Formula I.

FIG. 16 is an X-ray powder diffraction pattern of an adipate crystal form 1 of a compound represented by Formula I.

FIG. 17 is a TGA pattern of an adipate crystal form 1 of a compound represented by Formula I.

FIG. 18 is a DSC pattern of an adipate crystal form 1 of a compound represented by Formula I.

FIG. 19 is a dynamic vapor sorption curve of an amorphous form of a compound represented by Formula I.

FIG. 20 is a dynamic vapor sorption curve of a crystal form A of a compound represented by Formula I.

FIG. 21 is a dynamic vapor sorption curve of a maleate crystal form 2 of a compound represented by Formula I.

FIG. 22 is a dynamic vapor sorption curve of an L-tartrate crystal form 1 of a compound represented by Formula I.

FIG. 23 is a dynamic vapor sorption curve of a phosphate crystal form 1 of a compound represented by Formula I.

Unless otherwise stated, the information about detection instruments and the parameters in detection methods used in the present disclosure are as described below.

Table 1

| Device name | X-ray powder diffractometer (XRPD) |
|---|---|
| Instrument | Bruker D8 Advance |
| Technical index | Cu Kα radiation with a wavelength of 1.54 Å (40 kV, 40 mA), θ-2θ goniometer, Ni monochromator, Lynxeye detector |
| Calibration substance | $Al_2O_3$ |

(continued)

| | | |
|---|---|---|
| Parameter | Detection angle | 3 - 40°2θ |
| | Step | 0.02°2θ |

Table 2

| | |
|---|---|
| Device name | Differential scanning calorimeter (DSC) |
| Instrument | Discovery DSC 2500 |
| Sample tray | Aluminum crucible |
| Protective gas | Nitrogen |
| Gas flowrate | 50 mL/min |
| Common detection method | Ramp 10 °C/min |

Table 3

| | |
|---|---|
| Device name | Thermogravimetric analyzer (TGA) |
| Instrument | Discovery TGA 550 |
| Sample tray | Platinum crucible + aluminum crucible |
| Protective gas | Nitrogen |
| Gas flowrate | 40 mL/min |
| Detection method | Ramp 10 °C/min |

Table 4

| | |
|---|---|
| Device name | Dynamic vapor sorption (DVS) meter |
| Manufacturer | Surface Measurement Systems |
| Instrument | DVS Resolution |
| Sample tray | Aluminum crucible |
| Protective gas | Nitrogen |
| Gas flowrate | 200 sccm |

Table 5

| | |
|---|---|
| Instrument | Nuclear magnetometer (NMR) |
| Model | Bruker Avance Neo 500 |
| Detection type | nuclear magnetic resonance hydrogen spectrum |
| Parameter | NS: 32<br>D1: 1 [sec] |
| | O1P: 6.175ppm<br>SW: 19.9955ppm<br>TE: 298K |

Table 6

| Device Name | High-performance liquid chromatograph (HPLC) |
|---|---|
| Manufacturer | Waters |
| Model | H-Class |
| Instrument | Constant-temperature and constant-humidity box |
| Model | Binder KBF720 constant-temperature and constant-humidity box |

**Method for detecting phosphoric acid content: Ion chromatography**

Instrument: Ion chromatograph

Chromatographic column: Dionex Ionpac® AS11-HC Analytical Column, 4.0×250 mm

Protective column: Dionex Ionpac® AG11-HC

Flowrate: 1.0 mL/min

Injection volume: 10 μL

Column temperature: 30 °C

Operating time: About 20 min

Rinsing solution: Potassium hydroxide solution of 25 mmol/L

Current: 62 mA

Diluent: Acetonitrile:water (50:50)

Specific test procedure: Operation under red light in a dark room.

Preparation of a control solution: About 25 mg of anhydrous monosodium phosphate was taken as control, accurately weighed, placed in a 100 mL volumetric flask, dissolved in water and diluted to a scale and shaken uniformly, and 1 mL was accurately measured and placed in a 10 mL volumetric flask, diluted with water to a scale and shaken uniformly so that the control solution was obtained.

Preparation of a sample solution: About 20 mg of the sample was accurately weighed, placed in a 200 mL brown volumetric flask, added with an appropriate amount of a diluent, sonicated to be dissolved, diluted with the diluent to a scale and shaken uniformly so that the sample solution was obtained.

Determination method: 10 μL of the control solution and 10 μL of the sample solution was accurately measured and injected into the ion chromatograph separately, and chromatograms were recorded. The content is calculated by external standard method using peak area..

Calculation formulas:

$$\mathrm{f} = \frac{W_{control} \times Content\ of\ the\ control \times 97.99}{A_{control} \times Dilution\ factor\ of\ the\ control \times 119.98}$$

$$\text{Phosphoric acid content } (\%) = \frac{A_{sample} \times f_{average} \times Dilution\ Factor\ of\ the\ sample}{W_{sample} \times (1 - Moisture\ in\ the\ sample)} \times 100$$

**Single crystal detection**

Single crystal data and Cu Kα radiation were collected at 150K using BRUKER D8 VENTURE; the crystal structure was analyzed by a direct method (Shelxs97), structural parameters were corrected and the types of atoms were discriminated by a method of least squares, and the positions of all hydrogen atoms were obtained by a geometric calculation method and a difference Fourier method.

DETAILED DESCRIPTION

The present disclosure is further described below by the examples given below, which do not constitute any limitation on the scope of the present disclosure. In the examples of the present disclosure, the techniques or methods, unless particularly stated otherwise, are conventional techniques or methods in the art. Unless particularly stated otherwise, the compound represented by Formula I as a starting material in the following Examples 2 to 10 is the compound represented by Formula I obtained with reference to the preparation method in Example 1. In the following examples, unless otherwise stated, the raw materials and reagents are commercially available; and the percentages, proportions, ratios or parts are calculated by weight.

Abbreviations

API: Active pharmaceutical ingredient;

DCM: Dichloromethane;

DMF: *N*,*N*-dimethylformamide;

EA: Ethyl acetate;

$CH_3OH$: Methanol;

$Cs_2CO_3$: Cesium carbonate;

hrs/h: Hours;

HPLC: High-performance liquid chromatography;

LCMS/LC-MS: Liquid chromatography-mass spectrometry;

lux: Lux;

min: Minutes;

mCPBA: Meta-chloroperoxybenzoic acid;

$Pd(PPh_3)_4$: Tetrakis(triphenylphosphine)palladium;

$Pd(OAc)_2$: Palladium acetate;

KOAc: Potassium acetate;

$K_2CO_3$: Potassium carbonate;

$Na_2CO_3$: Sodium carbonate;

NaH: Sodium hydride;

$PdCl_2(dppf)CH_2Cl_2$: [1,1'-Bis(diphenylphosphine)ferrocene]dichloropalladium dichloromethane complex;

Xant-phos: 4,5-Bis(diphenylphosphino)-9,9-dimethylxanthene;

RH: Relative humidity;

RRT: Relative retention time;

TEA: Triethylamine;

$^1$H-NMR/HNMR: Nuclear magnetic resonance hydrogen spectrum;

XRD/XRPD: X-ray powder diffraction;

DSC: Differential scanning calorimetry;

TGA: Thermogravimetric analysis;

DVS: Dynamic vapor sorption.

**Synthesis of Intermediate 1-S4**

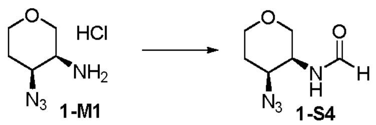

3.57 g of 1-M1, 6.2 mL of TEA and 2.09 g of formic acid (content: 88%) were dissolved in 100 mL of ethyl formate, heated and refluxed for 4 hrs. It was detected through LCMS that the reaction was complete. The system was concentrated under reduced pressure, the residue was dissolved in ethyl acetate, and an organic layer was washed with a small amount of water and saturated brine in sequence and concentrated to obtain 3.49 g of Compound 1-S4, which was directly used in the next step without purification.

LC-MS [M+H$^+$]: 171.08.

**Example 1 Synthesis of a compound represented by Formula I**

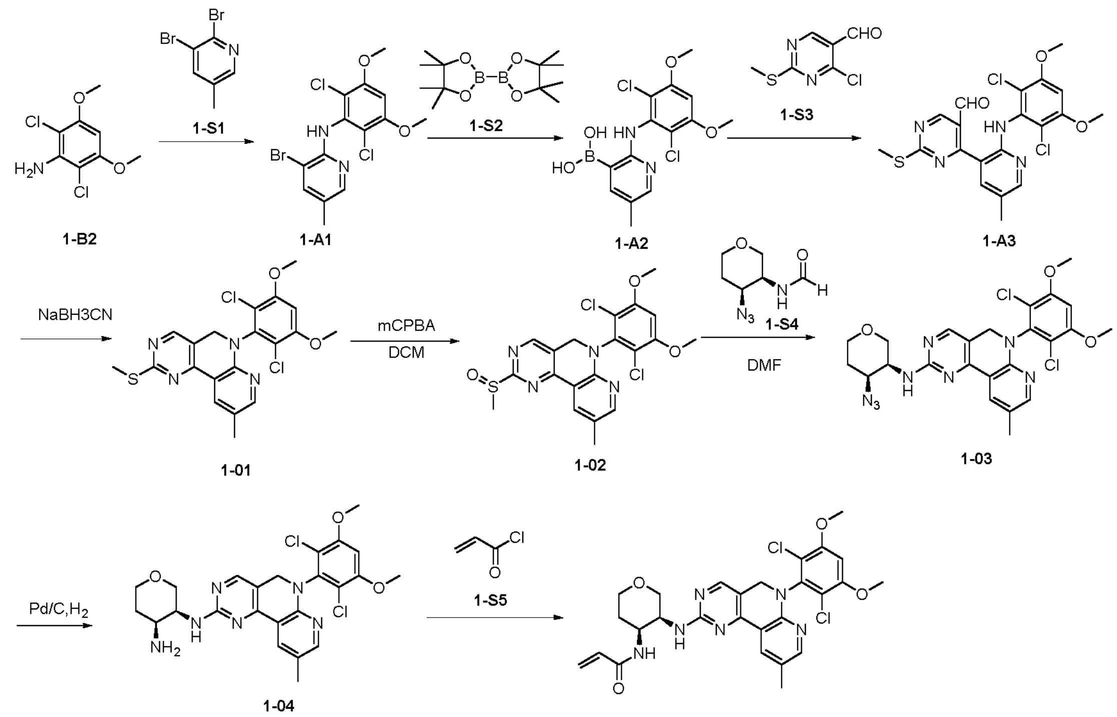


**Step 1: Preparation of Compound 1-A1**

15.0 g of 1-B2, 17.80 g of 1-S1, 44.02 g of $Cs_2CO_3$, 1.52 g of $Pd(OAc)_2$ and 5.86 g of Xant-phos were dissolved in 500 mL of toluene and reacted at 115 °C for 8 hrs under nitrogen protection. It was detected through LCMS that the reaction was complete. The system was concentrated under reduced pressure, and the residue was dissolved in water and extracted with DCM. The organic layer was washed with water and saturated brine in sequence and concentrated, and the residue was isolated and purified by column chromatography with n-hexane:dichloromethane (5:1) as eluents to obtain Compound 1-A1.

LC-MS [$M+H^+$]: 390.95.

**Step 2: Preparation of Compound 1-A2**

21.91 g of 1-A1, 21.29 g of 1-S2, 16.46 g of KOAc and 3.66 g of $PdCl_2(dppf)CH_2Cl_2$ were dissolved in 340 mL of 1,4-dioxane and reacted at 100 °C for 8 hrs under nitrogen protection. It was detected through LCMS that the reaction was complete. The system was concentrated under reduced pressure, the residue was added with a mixture of water and EA, stirred, dispersed and filtered, and the filter cake was dried to obtain Compound 1-A2.

LC-MS [$M+H^+$]: 357.05.

**Step 3: Preparation of Compound 1-A3**

15.84 g of 1-A2, 9.21 g of 1-S3, 12.27 g of $K_2CO_3$ and 5.12 g of $Pd(PPh_3)_4$ were dissolved in 350 mL of 1,4-dioxane/40 mL of water and reacted at 90 °C for 6 hrs under nitrogen protection. It was detected through LCMS that the reaction was complete. The system was concentrated under reduced pressure, and the residue was dissolved in water and extracted with DCM. The organic layer was washed with water and saturated brine in sequence and concentrated, and the residue was isolated and purified by column chromatography with dichloromethane:methanol (200:1) as eluents to obtain Compound 1-A3.

LC-MS [$M+H^+$]: 465.05.

**Step 4: Preparation of Compound 1-01**

8.50 g of 1-A3, 1.65 g of glacial acetic acid and 1.72 g of cyano sodium borohydride were dissolved in a mixed solution of 100 mL of methanol and 150 mL of dichloromethane and reacted at room temperature for 4 hrs under nitrogen protection. It was detected through LCMS that the reaction was complete. The system was concentrated under reduced pressure and the residue was added with an aqueous solution of $Na_2CO_3$ to be dispersed and dissolved. The resulting

mixture was extracted twice with DCM and the organic phases were combined. The organic phases were washed with water, dried and filtered, and the filtrate was concentrated under reduced pressure to obtain Compound 1-01.

LC-MS [M+H$^+$]: 449.05.

**Step 5: Preparation of Compound 1-02**

7.17 g of 1-01 was dissolved in 200 mL of DCM, and 10.37 g of mCPBA (content: 85%) was added slowly in an ice-water bath. After addition, the system was naturally warmed to room temperature and reacted for 4 hrs. It was detected through LCMS that the reaction was complete. The reaction solution was washed twice with a saturated aqueous solution of sodium bicarbonate, the organic layer was washed with water and saturated brine in sequence, dried over anhydrous sodium sulfate, filtered and concentrated, and the residue was isolated and purified by column chromatography with dichloromethane as an eluent to obtain Compound 1-02.

LC-MS [M+H$^+$]: 465.05.

**Step 6: Preparation of Compound 1-03**

2.83 g of 1-S4 was dissolved in 20 mL of DMF and cooled to 0 °C, and 0.77 g of sodium hydride (content: 60%) was slowly added and reacted at this temperature for 40 min. 10 mL of a solution of Compound 1-02 (6.18 g) in DMF was added and stirred for 40 min, the reaction was quenched by adding DCM and water to the reaction solution, layers were separated, and the aqueous layer was extracted twice with DCM. The combined organic layer was washed with water and saturated brine in sequence, isolated and concentrated, and the residue was isolated and purified by column chromatography with dichloromethane:methanol (100:1) as eluents to obtain Compound 1-03.

LC-MS [M+H$^+$]: 543.13.

**Step 7: Preparation of Compound 1-04**

1.29 g of Compound 1-03 and 0.52 g of 10% Pd/C were dissolved in a mixed solvent of 40 mL of EA and 40 mL of $CH_3OH$ and heated to 30 °C and reacted for 2 hrs under $H_2$ protection. The system was filtered and concentrated under reduced pressure, and the residue was isolated and purified by column chromatography with dichloromethane:methanol (40:1) as eluents to obtain Compound 1-04.

LC-MS [M+H$^+$]: 517.14.

**Step 8: Preparation of the compound represented by Formula I (free bases (FB))**

1.77 g of Compound 1-04 was dissolved in 40 mL of DCM, 1.90 mL of triethylamine was added and cooled to -20 °C, and 0.31 g of acryloyl chloride was slowly added dropwise and reacted at this temperature for 1 h. It was detected through LCMS that the reaction was complete. The reaction solution was quenched with a saturated aqueous solution of $NaHCO_3$, the organic layer was washed with water and saturated brine in sequence and concentrated under reduced pressure, and the residue was isolated and purified by column chromatography with DCM:MeOH (80:1) as eluents to obtain the compound represented by Formula I.

LC-MS [M+H$^+$]: 571.15.

$^1$H NMR (500 MHz, DMSO-$d_6$): $\delta$(ppm) 8.15 (s, 1H), 8.12 (s, 1H), 8.00 (d, *J* = 7.8 Hz, 1H), 7.79 (s, 1H), 6.96 (s, 1H), 6.59 (d, *J* = 7.5 Hz, 1H), 6.20 (br, 1H), 6.02-6.05 (m, 1H), 5.49 (br, 1H), 4.76 (d, *J* = 14.0 Hz, 1H), 4.71 (d, *J* = 14.0 Hz, 1H), 4.45 (br, 1H), 4.24 (br, 1H), 3.96 (s, 6H), 3.81-3.87 (m, 2H), 3.62-3.64 (m, 1H), 3.51-3.64 (m, 1H), 2.17(s, 3H), 1.92-1.99 (m, 1H), 1.63-1.65 (m, 1H).

**Example 2 Screening of crystalline salts of a compound represented by Formula I**

The crystalline salts of the compound represented by Formula I were screened and the experiments were carried out by feeding the compound represented by Formula I and ions in different solvent systems, as detailed in Table 7.

Table 7 Summary of data of the screening experiments on salt forms

| Salt | Feeding Molar Ratio (Free Base:Acid) | solvent | Result |
|---|---|---|---|
| Maleic acid | 1:1.1 | Acetone/methyl t-butyl ether | Crystalline salt |

(continued)

| Salt | Feeding Molar Ratio (Free Base:Acid) | solvent | Result |
|---|---|---|---|
| L-tartaric acid | 1:1.1 | Acetone/methyl t-butyl ether | Crystalline salt |
| Phosphoric acid | 1:1.1 | Acetone | Crystalline salt |
| Benzenesulfonic acid | 1:1.1 | Acetone/methyl t-butyl ether | No crystalline salt formed |
| Methanesulfonic acid | 1:1.1 | Acetone/methyl t-butyl ether | Mesylate obtained in a weakly crystalline state |
| Sulfuric acid | 1:1.1 | Acetone | No crystalline salt formed |
| P-toluenesulfonic acid | 1:1.1 | Acetone/methyl t-butyl ether | No crystalline salt formed |
| Aspartic acid | 1:1.1 | Acetone | No salt formed |
| Citric acid | 1:1.1 | Acetone/methyl t-butyl ether | No salt formed |
| L-malic acid | 1:1.1 | Acetone/methyl t-butyl ether | No salt formed |
| Fumaric acid | 1:1.1 | Acetone/methyl t-butyl ether | No salt formed |
| Oxalic acid | 1:1.1 | Acetone/methyl t-butyl ether | No salt formed |

In the presence of benzenesulfonic acid, sulfuric acid, p-toluenesulfonic acid, aspartic acid, citric acid, L-malic acid, fumaric acid and oxalic acid, the formation of the crystalline salts of the compound represented by Formula I was not observed under experimental conditions. In the presence of methanesulfonic acid, maleic acid, tartaric acid and phosphoric acid, the formation of the crystalline salts of the compound represented by Formula I was observed, but the crystallization of the mesylate shows a weakly crystalline state.

**Example 3 Preparation method for a crystal form A of a compound represented by Formula I**

8.3 g of the compound represented by Formula I was added with 80 mL of ethyl acetate, pulped for 15 hrs and suction filtered under reduced pressure, and the filter cake was dried under reduced pressure at 50 °C for 6 hours in a vacuum drying oven to obtain 7.10 g of a yellow solid, the crystal form A of the compound represented by Formula I.

**Example 4 Preparation method for a crystal form A of a compound represented by Formula I**

A solution of the compound represented by Formula I (1.5 kg) in dichloromethane (16 L) was concentrated under reduced pressure, and the crystal form A of the compound represented by Formula I was slowly added to the concentrated system as a seed (10.01 g of crystal form A was dispersed in 500 mL of a suspension system of ethyl acetate). Ethyl acetate (12 L) was slowly added with the continued concentration under reduced pressure. The concentration was stopped when the remaining amount of solvent was about 6-7 L. The resulting concentrated system was transferred to a 20 L reaction kettle and stirring was continued for 12 hrs. The concentrated system was filtered, and the resulting solid was placed in a vacuum drying oven and dried in vacuum at 50 °C for 6 to 18 hrs. The solid was weighed to obtain 0.84 kg of the crystal form A of the compound represented by Formula I.

$^1$H NMR (500 MHz, DMSO-$d_6$): $\delta$(ppm) 8.15 (s, 1H), 8.12 (s, 1H), 8.00 (d, $J$ = 7.8 Hz, 1H), 7.79 (s, 1H), 6.96 (s, 1H), 6.59 (d, $J$ = 7.5 Hz, 1H), 6.20 (br, 1H), 6.02-6.05 (m, 1H), 5.49 (br, 1H), 4.76 (d, $J$ = 14.0 Hz, 1H), 4.71 (d, $J$ = 14.0 Hz, 1H), 4.45 (br, 1H), 4.24 (br, 1H), 3.96 (s, 6H), 3.81-3.87 (m, 2H), 3.62-3.64 (m, 1H), 3.51-3.64 (m, 1H), 2.17(s, 3H), 1.92-1.99 (m, 1H), 1.63-1.65 (m, 1H).

The main data of the X-ray powder diffraction pattern of the crystal form A of the compound represented by Formula I is shown in Table 8.

Table 8

| No. | 2θ±0.2(°) | Interplanar Spacing [Å] | Relative Intensity (%) |
|---|---|---|---|
| 1 | 7.5 | 11.771 | 100.0 |
| 2 | 8.1 | 10.932 | 7.8 |
| 3 | 9.1 | 9.721 | 9.0 |
| 4 | 10.1 | 8.726 | 14.0 |
| 5 | 10.4 | 8.511 | 19.9 |
| 6 | 10.7 | 8.229 | 93.8 |
| 7 | 11.3 | 7.798 | 68.9 |
| 8 | 13.1 | 6.763 | 58.1 |
| 9 | 15.1 | 5.878 | 86.5 |
| 10 | 16.0 | 5.549 | 17.5 |
| 11 | 17.5 | 5.054 | 18.8 |
| 12 | 19.3 | 4.585 | 13.2 |
| 13 | 19.9 | 4.468 | 13.9 |
| 14 | 20.4 | 4.356 | 27.6 |
| 15 | 20.9 | 4.249 | 80.5 |
| 16 | 21.6 | 4.108 | 44.1 |
| 17 | 22.2 | 4.006 | 18.5 |
| 18 | 22.4 | 3.961 | 26.6 |
| 19 | 22.8 | 3.895 | 39.3 |
| 20 | 23.5 | 3.788 | 53.8 |
| 21 | 24.0 | 3.703 | 21.2 |
| 22 | 24.5 | 3.628 | 13.6 |
| 23 | 24.8 | 3.591 | 12.5 |
| 24 | 25.2 | 3.525 | 63.6 |
| 25 | 28.2 | 3.160 | 7.8 |
| 26 | 30.2 | 2.958 | 49.9 |

**Example 5 Preparation method for a phosphate crystal form 1 of a compound represented by Formula I**

Isopropanol (608 mL) and the compound represented by Formula I (34.20 g) were added to a four-necked flask in sequence, stirred and heated. After the system was warmed to 72 °C, 20.70 g of an aqueous solution of phosphoric acid (85% phosphoric acid dissolved in 86 mL of purified water) was slowly added dropwise. After the addition, the system was warmed to 81 °C and completely dissolved. The system was filtered, and the filtrate was stirred at 80 °C for 20 min, cooled to room temperature and stirred overnight. Then, n-heptane (628 mL) was added dropwise to the reaction system and stirred for 6 hrs. The reaction solution was filtered and the filter cake was pumped to dryness and dried in vacuum at 60 °C for 12 hrs to obtain 41.49 g of the phosphate crystal form 1 of the compound represented by Formula I. The XRPD pattern is shown in FIG. 9.

**Example 6 Preparation method for a phosphate crystal form 1 of a compound represented by Formula I**

The compound represented by Formula I (700.38 g, 1.23 mol), isopropanol (11 L) and purified water (1.58 L) were added to a reaction kettle, and the mixture was stirred and warmed. When the inner temperature was 77 °C, an

aqueous solution of 85% phosphoric acid (423.87 g, 3.68 mol) was slowly added dropwise. After 45 min, the addition was completed, the reaction was continued with the temperature maintained, and the system was completely clarified at 80 °C. The materials were discharged and filtered when the system was still warm. The filtrate was added again to the reaction kettle. The reaction was heated to 80 °C, an oil bath was removed, and the system was naturally cooled. When the system was cooled to 65 °C, 3.5 g of phosphate crystal form 1 was added as a seed. When the system was cooled to about 45 °C, the solid was gradually precipitated. After 12 h, n-heptane (11 L) was added dropwise. The addition was completed after 2 h. The system was stirred for 6 h and suction-filtered, and the filter cake was dried in vacuum at 60 °C to obtain 766.21 g of the phosphate crystal form 1 of the compound represented by Formula I. The XRPD pattern is shown in FIG. 10.

$^1$H NMR (500 MHz, DMSO): $\delta$ 8.15 (s, 1H), 8.12 (s, 1H), 8.00 (d, *J* = 7.8 Hz, 1H), 7.79 (s, 1H), 6.96 (s, 1H), 6.59 (d, *J* = 8.0 Hz, 1H), 6.20 (s, 1H), 6.03 (d, *J* = 15.0 Hz, 1H), 5.49 (s, 1H), 4.74 (q, *J* = 14.1 Hz, 2H), 4.45 (s, 1H), 4.27 - 4.19 (m, 1H), 3.96 (s, 6H), 3.87 - 3.81 (m, 2H), 3.64 - 3.62 (m, 1H), 3.54 - 3.51 (m, 1H), 2.17 (s, 3H), 1.99 - 1.92 (m, 1H), 1.65 - 1.63 (m, 1H).

The main data of the X-ray powder diffraction pattern of the phosphate crystal form 1 of the compound represented by Formula I is shown in Table 9.

Table 9

| No. | 2θ ± 0.2 (°) | Interplanar Spacing [Å] | Relative Intensity (%) |
|---|---|---|---|
| 1 | 4.9 | 17.944 | 59.9 |
| | 7.9 | 11.203 | 13.8 |
| 2 | 8.3 | 10.693 | 50.1 |
| 3 | 9.3 | 9.509 | 11.9 |
| 4 | 9.9 | 8.970 | 16.7 |
| 5 | 10.7 | 8.229 | 46.8 |
| 6 | 11.6 | 7.654 | 38.0 |
| 7 | 12.8 | 6.907 | 48.2 |
| 8 | 13.2 | 6.688 | 21.7 |
| 9 | 13.5 | 6.573 | 21.0 |
| 10 | 14.1 | 6.268 | 11.2 |
| 11 | 14.4 | 6.152 | 23.6 |
| 12 | 14.9 | 5.920 | 11.4 |
| 14 | 16.8 | 5.275 | 100.0 |
| 15 | 19.8 | 4.480 | 19.4 |
| 16 | 20.1 | 4.415 | 75.5 |
| 17 | 20.6 | 4.315 | 37.7 |
| 18 | 21.3 | 4.167 | 98.5 |
| 19 | 21.6 | 4.111 | 65.6 |
| 20 | 22.8 | 3.889 | 45.7 |
| 21 | 24.7 | 3.596 | 22.8 |
| 22 | 28.5 | 3.133 | 43.5 |
| 23 | 29.9 | 2.989 | 13.6 |

The phosphate radical content was detected by ion chromatography (where the same sample was detected twice). Theoretical value of 1.5 phosphoric acid contents: 20.46%, ±10% range: 18.41%-22.50%. The detection results are shown in Table 10.

Table 10

| Detected Raw Material | Content (%) | Average Content (%) |
|---|---|---|
| Phosphate crystal form 1 | 20.88 | 20.77 |
| | 20.66 | |

The unit cell parameters of the phosphate crystal form 1 are described below.

The crystal belongs to a monoclinic system with a space group being $P2_1$,

a = 8.4064(17),

b = 35.714(7),

c = 11.122(2) Å,

$\alpha = \gamma = 90.00°$,

$\beta = 91.14(3)°$;

cell volume V = 3338.5(12) $Å^3$;

the number of asymmetric units in a unit cell Z = 2.

**Example 7 Preparation method for a maleate crystal form 1 of a compound represented by Formula I**

500 mg of the compound represented by Formula I was added to 10 mL of acetone and stirred and dissolved in a water bath of 40 °C. 112 mg of maleic acid was dissolved in 0.5 mL of acetone, added dropwise to the above system at room temperature, warmed to 50 °C and stirred for 20 min to precipitate a solid. The reaction solution was naturally cooled to room temperature and stirred for 14 h. The system was filtered, and the filter cake was rinsed with 5 mL of acetone and dried in vacuum at room temperature to obtain 400 mg of the maleate crystal form 1 of the compound represented by Formula I.

$^1$H NMR (500 MHz, DMSO) $\delta$ 8.15 (s, 1H), 8.12 (s, 1H), 7.98 (d, J = 7.8 Hz, 1H), 7.79 (s, 1H), 6.96 (s, 1H), 6.65 (s, 1H), 6.25 (s, 2H), 6.18 (s, 1H), 6.03 (d, J = 15.0 Hz, 1H), 5.48 (s, 1H), 4.74 (q, J = 14.1 Hz, 2H), 4.45 (s, 1H), 4.27-4.19 (m, 1H), 3.95 (s, 6H), 3.90-3.74 (m, 2H), 3.68-3.57 (m, 1H), 3.56-3.48 (m, 1H), 2.17 (s, 3H), 2.00-1.88 (m, 1H), 1.68-1.58 (m, 1H).

The main data of the X-ray powder diffraction pattern of the maleate crystal form 1 of the compound represented by Formula I is shown in Table 11.

Table 11

| No. | $2\theta \pm 0.2$ (°) | Relative Intensity (%) |
|---|---|---|
| 1 | 3.7 | 57.9 |
| 2 | 9.7 | 60.7 |
| 3 | 9.9 | 71.5 |
| 4 | 11.0 | 93.5 |
| 5 | 11.7 | 44.9 |
| 6 | 13.4 | 53.6 |
| 7 | 14.4 | 11.5 |
| 8 | 16.2 | 100.0 |
| 9 | 17.2 | 47.6 |
| 10 | 18.7 | 42.7 |

(continued)

| No. | 2θ±0.2 (°) | Relative Intensity (%) |
|---|---|---|
| 11 | 19.2 | 26.6 |
| 12 | 19.4 | 56.0 |
| 13 | 19.9 | 12.1 |
| 14 | 20.6 | 68.9 |
| 15 | 20.9 | 25.6 |
| 16 | 21.8 | 33.1 |
| 17 | 22.6 | 56.9 |
| 18 | 23.5 | 15.3 |
| 19 | 24.4 | 57.2 |
| 20 | 26.4 | 26.1 |

**Example 8 Preparation method for a maleate crystal form 2 of a compound represented by Formula I**

600 mg of the compound represented by Formula I was added to 20 mL of acetone, stirred and dissolved in a water bath of 50 °C, filtered and cooled to room temperature. 134.2 mg of maleic acid was dissolved in 1 mL of acetone, stirred at room temperature, added dropwise to the above system, and stirred at room temperature for 40 min. 54 mL of methyl t-butyl ether was added to the reaction solution and stirred overnight at 4 °C to precipitate a solid. The solid was suction filtered under reduced pressure and dried in vacuum at room temperature to obtain 486 mg of the maleate crystal form 2 of the compound represented by Formula I.

**Example 9 Preparation method for an L-tartrate crystal form 1 of a compound represented by Formula I**

120 mg of the compound represented by Formula I was added to 4 mL of acetone, stirred at 50 °C, filtered and cooled to room temperature. 34.7 mg of L-tartaric acid was dissolved in 1 mL of acetone, added dropwise to the above clear system and stirred for 30 min. 16 mL of methyl t-butyl ether was added dropwise and stirred overnight at 4 °C to precipitate a solid. The solid was suction filtered under reduced pressure and dried in vacuum at room temperature to obtain 106 mg of the L-tartrate crystal form 1 of the compound represented by Formula I.

The main data of the X-ray powder diffraction pattern of the L-tartrate crystal form 1 is shown in Table 12.

Table 12

| No. | 2θ±0.2(°) | Interplanar Spacing [Å] | Relative Intensity (%) |
|---|---|---|---|
| 1 | 5.4 | 16.370 | 100.0 |
| 2 | 12.9 | 6.845 | 23.0 |
| 3 | 13.6 | 6.513 | 10.4 |
| 4 | 15.4 | 5.757 | 11.3 |
| 5 | 16.1 | 5.501 | 13.5 |
| 6 | 16.5 | 5.374 | 42.6 |
| 7 | 17.6 | 5.024 | 74.6 |
| 8 | 19.7 | 4.502 | 62.8 |
| 9 | 20.1 | 4.409 | 21.8 |
| 10 | 20.7 | 4.287 | 53.5 |
| 11 | 21.8 | 4.065 | 15.6 |
| 12 | 22.2 | 3.997 | 34.6 |
| 13 | 22.6 | 3.931 | 27.1 |

(continued)

| No. | 2θ±0.2(°) | Interplanar Spacing [Å] | Relative Intensity (%) |
|---|---|---|---|
| 14 | 26.4 | 3.374 | 23.3 |

**Example 10 Preparation method for an adipate crystal form 1 of a compound represented by Formula I**

206 mg of the compound represented by Formula I and 63.3 mg of adipic acid were added to a 5 mL glass bottle, and 2.5 mL of ethyl acetate was added, stirred at room temperature for about 2.5 days, centrifuged to be isolated and dried by air blowing at 40 °C to obtain the adipate crystal form 1 of the compound represented by Formula I.

The main data of the X-ray powder diffraction pattern of the adipate crystal form 1 is shown in Table 13.

Table 13

| No. | 2θ± 0.2(°) | Interplanar Spacing [Å] | Relative Intensity (%) |
|---|---|---|---|
| 1 | 5.8 | 15.236 | 62.9 |
| 2 | 8.4 | 10.476 | 74.6 |
| 3 | 10.0 | 8.816 | 30.7 |
| 4 | 10.4 | 8.475 | 35.7 |
| 5 | 12.3 | 7.196 | 97.0 |
| 6 | 13.1 | 6.769 | 10.3 |
| 7 | 14.7 | 6.040 | 17.1 |
| 8 | 17.5 | 5.071 | 49.9 |
| 9 | 20.1 | 4.407 | 30.1 |
| 10 | 20.9 | 4.243 | 26.5 |
| 11 | 22.9 | 3.883 | 100.0 |
| 12 | 23.4 | 3.804 | 13.8 |
| 13 | 25.4 | 3.500 | 44.1 |
| 14 | 25.9 | 3.444 | 43.7 |
| 15 | 26.4 | 3.379 | 17.6 |
| 16 | 27.6 | 3.229 | 14.5 |

**Example 11 Hygroscopicity determination of crystal forms of a compound represented by Formula** I

The hygroscopicity of samples (which are multiple crystal forms shown in Table 14) was determined by a dynamic vapor sorption (DVS) meter. The samples were detected for changes of weight gains due to vapor absorption within the range of 0%RH (relative humidity) to 80%RH. The detection results are shown in FIGS. 19 to 23. The detection results are shown in Table 14.

Table 14 Changes of samples in weight within the range of 0%RH to 80%RH

| Crystal form | Weight Gain for Vapor Absorption |
|---|---|
| Amorphous form of the compound represented by Formula I | 6.53% |
| Crystal form A | 0.03% |
| Maleate crystal form 2 | 6.64% |
| L-tartrate crystal form 1 | 0.07% |
| Phosphate crystal form 1 | 0.19% |

As can be seen from the detection results, the crystal form A, L-tartrate crystal form 1 and phosphate crystal form 1 of the compound represented by Formula I are non-hygroscopic under the test conditions and have significant advantages.

**Example 12 Stability determination of crystal forms of a compound represented by Formula I**

Samples and experimental preparation: The amorphous form of the compound represented by Formula I and the crystal form A, maleate crystal form 1, maleate crystal form 2, phosphate crystal form 1 and adipate crystal form 1 of the compound represented by Formula I in appropriate amounts were tested for stability. Samples were taken at different time points separately for HPLC. The experimental conditions and the detection results are shown in Table 15. The packages were polyester/aluminum/polyamide/polyethylene pharmaceutical composite films and bags.
The HPLC detection results show that the crystal form A, maleate crystal form 1, phosphate crystal form 1 and adipate crystal form 1 of the compound represented by Formula I, which are provided in the present disclosure, have relatively good stability.

**Table 15**

| Sample | Test Condition | Sampling Time/Day | Changed Maximum Single Impurity/% | API Content/ % |
|---|---|---|---|---|
| Amorphous form of the compound represented by Formula I | - | 0 | 0.41 | 98.30 |
| | 40 °C, open | 10 | 1.15 | 97.45 |
| | 25 °C/75%RH, open | 10 | 1.26 | 97.21 |
| Maleate crystal form 2 | - | 0 | 0.29 | 99.63 |
| | 40 °C, open | 10 | 0.48 | 99.32 |
| | 25 °C/75%RH, open | 10 | 1.1 | 98.61 |
| Crystal form A of the compound represented by Formula I | - | 0 | 0.19 | 99.16 |
| | 60 °C, open | 12 | 0.24 | 99.14 |
| | 25 °C/92.5%R H, open | 20 | 0.17 | 99.32 |
| | 40 °C/75%RH, packaged | 5 | 0.19 | 99.25 |
| | | 20 | 0.17 | 99.47 |
| Phosphate crystal form 1 | - | 0 | 0.15 | 99.32 |
| | 40 °C/75%RH, packaged | 5 | 0.15 | 99.39 |
| | | 12 | 0.11 | 99.56 |
| Adipate crystal form 1 | - | 0 | 0.15 | 99.38 |
| | 40 °C/75%RH, packaged | 5 | 0.16 | 99.35 |
| | | 12 | 0.15 | 99.45 |
| Maleate crystal form 1 | - | 0 | 0.2 | 99.62 |
| | 40 °C/75%RH, packaged | 5 | 0.26 | 99.48 |
| | | 12 | 0.28 | 99.34 |

The phosphate and maleate of the compound represented by Formula I have unexpected significant advantages over other salts, such as high physical and chemical stability. In the salt form screening process, the formation of the crystalline salts of the compound represented by Formula I was not observed in the presence of benzenesulfonic acid, sulfuric acid, p-toluenesulfonic acid, aspartic acid, citric acid, L-malic acid, fumaric acid and oxalic acid. Further, the stable crystal form A, maleate crystal form 1 and phosphate crystal form 1 of the compound represented by Formula I

have surprising advantages. For example, the crystal form A, maleate crystal form 1 and phosphate crystal form 1 of FBs have low hygroscopicity and their crystalline states are not easy to change with ambient temperature and humidity. In contrast, the maleate crystal form 2 and the amorphous form of the compound represented by Formula I are hygroscopic under relative humidity and have poor stability so that they are easy to change in weight and/or phase.

**Example 13 Pharmacokinetic test of crystal forms of a compound represented by Formula I**

Drug preparation: A maleate crystal form 1, a phosphate crystal form 1 and an adipate crystal form 1 were weighed separately, and each crystal form was prepared in a 0.5% CMC-Na solution into a to-be-tested suspension, where the final concentration of each compound was 5 mg/mL.

Test animals: SD rats were randomly divided into three groups, each group including three male rats, and the three groups were called a maleate group, a phosphate group and an adipate group, separately.

Administration and sample collection: The SD rats in each group were subjected to oral administration at a dosage of 50 mg/kg. Blood collection time: Blood samples were collected in EDTA-K pre-anticoagulation tubes after 0 h (before administration), 0.25 h, 0.5 h, 1 h, 2 hrs, 4 hrs, 7 hrs and 24 hrs. The plasma in each sample was isolated by being centrifuged at 4000 rpm for 10 min at 4 °C. Plasma samples were collected and stored at -80 °C for analytical use. The concentration of a to-be-tested compound in plasma samples was analyzed by AB4000 API LC/MS in combination with HPLC, and the pharmacokinetic parameter of the to-be-tested compound was obtained by analyzing the plasma concentration-time data of each animal. The data is shown in Table 16.

Table 16

| Crystal form | AUClast (h*ng/mL) | Cmax (ng/mL) (ng/mL*h) |
|---|---|---|
| Adipate crystal form 1 of the compound | 3897 | 584 |
| represented by Formula I | | |
| Maleate crystal form 1 of the compound represented by Formula I | 8355 | 2713 |
| Phosphate crystal form 1 of the compound represented by Formula I | 6894 | 1137 |

**Example 14 Pharmacological test of a compound represented by Formula I**

**Example A Kinase assay**

The effect of the compound represented by Formula I of the present disclosure on the activity of tyrosine kinase FGFR4 was evaluated by an *in vitro* kinase detection experiment. Mobility shift assay was used in the experiment, and a fluorescently labeled polypeptide was used as a substrate. The substrate was converted into a product under the action of an enzyme in the reaction system and its charge also changed accordingly. This method can isolate the substrate from the product by using a difference between their charges and then detect the substrate and the product separately.

Experimental procedures

(1) Compound preparation

A DMSO solution of the compound (300 μM) was diluted to a DMSO solution with a 100-fold final concentration in a 384-well plate through 3-fold gradient dilution, and 250 nL of the compound with a 100-fold final concentration was transferred to a target plate OptiPlate-384F by a dispenser Echo 550. The final concentrations of the compound were 3000 nM, 1000 nM, 333.3 nM, 111.1 nM, 37.04 nM, 12.35 nM, 4.115 nM, 1.372 nM, 0.4572 nM and 0.1524 nM. The compound and the enzyme were incubated for 60 min.

(2) Kinase reaction

A 1× kinase buffer was prepared and used for preparing a kinase solution with a 2.5-fold final concentration, 10 μL of the kinase solution with a 2.5-fold final concentration was added to a compound well and a positive control well separately, and 10 μL of 1× kinase buffer was added to a negative control well. After centrifugation, the reaction plate

was shaken, mixed uniformly and incubated at room temperature for 60 min. A mixture of adenosine triphosphate (ATP) and kinase substrate 22 with a 25/15-fold final concentration was prepared with the 1× kinase buffer. 15 μL of the mixture of ATP and the substrate with a 25/15-fold final concentration was added to start the reaction, and the 384-well plate was centrifuged, shaken, mixed uniformly and incubated at room temperature for 30 min. 30 μL of a stop detection solution was added to stop the kinase reaction, centrifuged, shaken and mixed uniformly, and a conversion rate was read with Caliper EZ Reader.

(3) Data analysis

$$\text{Calculation formula: inhibition rate (\%)} = \frac{\text{Conversion\%}_{\text{max}} - \text{Conversion\%_sample}}{\text{Conversion\%}_{\text{max}} - \text{Conversion\%_min}} * 100$$

In the formula, Conversion%_sample denotes the conversion rate of the sample; Conversion%_min denotes the average value of the negative control well, which represents the conversion rate of a well without enzyme activity; Conversion%_max denotes the average value of the positive control well, which represents the conversion rate of a well without compound inhibition.

With a log value of the concentration as an X axis and a percentage inhibition rate as a Y axis, a dose-response curve was fitted using log(inhibitor) vs. response-Variable slope (four parameters) of GraphPad Prism analysis software so that the $IC_{50}$ value of each compound on enzyme activity was obtained. The formula is as follows: Y = Bottom + (Top - Bottom)/(1+10 ∧ (($\log IC_{50}$ - X)∗HillSlope)).

The obtained $IC_{50}$ data is shown in Table 17.

Table 17

| Compound | Compound $IC_{50}$ (nM) | |
|---|---|---|
| | FGFR1 | FGFR4 |
| Compound represented by Formula I | 367 | 1.0 |
| BLU554(1) | 1480 | 13 |

Note: (1) BLU554 is Compound No. 40 disclosed by Blueprint Medicines Corporation in WO2015061572.

The compound of the present disclosure has an inhibitory effect on FGFR4 kinase, and such compounds have a much stronger inhibitory effect on FGFR4 than FGFR1, which represents very good selectivity.

**Example B Cell proliferation assay**

The effect of the compound of the present disclosure on the proliferation of human liver cancer cells Hep3B cells was evaluated by an *in vitro* cell assay. CELL TITER-GLO (CTG) luminescence was used as a detection method in the assay, which can detect the number of living cells by quantitatively determining ATP. Since ATP participates in a variety of enzymatic reactions in organisms, the ATP is an indicator of the metabolism of living cells and its content directly reflects the number of cells and the cell state of cells. In the assay, CellTiter-Glo™ reagent was added to a cell culture medium to measure a luminescence value, the luminescence value is directly proportional to the amount of ATP, and ATP is positively correlated to the number of living cells, so cell viability can be inspected by detecting the ATP content.

Experimental procedures

(1) Cell plating

A bottle of Hep3B cells in a logarithmic growth phase was taken, the cells were counted after digestion and resuspension and then inoculated into a 96-well plate after the cell density was adjusted, each well inoculated with 180 μL (1500 cells/well), and the plate was incubated for 24 hrs in a 37 °C, 5% $CO_2$ incubator.

(2) Administration to cells

A test substance (600 μM) dissolved in DMSO was gradient diluted with DMSO at a ratio of 1:3 to a solution with a 200-fold final concentration, then a cell culture solution was diluted by 20 fold (10×), and 20 μL of the compound solution was added to a 96-well plate containing cells. The final concentrations of the compound from high to low were 3000 nM, 1000 nM, 333.3 nM, 111.1 nM, 37.04 nM, 12.35 nM, 4.115 nM, 1.372 nM and 0.4572 nM. The plate was placed in a 37 °C, 5% $CO_2$ incubator and incubated for 96 hrs.

(3) CTG detection

After 96 hrs of incubation, 60 μL of CellTiter-Glo® Luminescent Cell Viability Assay solution was added to each well, gently shaken for 2 min and incubated for 10 min at room temperature, and the luminescence value of each well on a multifunctional microplate reader was read.

(4) Data analysis

An inhibition rate was calculated according to the luminescence value.

Inhibition rate (%) = (blank group value – administration group value)/(blank group value – zero-adjustment group value) * 100

With a log value of the concentration as an X axis and a percentage inhibition rate as a Y axis, a dose-response curve was fitted using log(inhibitor) vs. response-Variable slope (four parameters) of GraphPad Prism, and the $IC_{50}$ of the compound in inhibiting cell proliferation was calculated.
The assay data is shown in Table 18.

Table 18

| Compound No. | $IC_{50}$ (nM) of the Compound on Hep3B Cells |
|---|---|
| BLU554 | 62.7 |
| Compound represented by Formula I | 5.1 |

The compound represented by Formula I of the present disclosure has a good inhibitory effect on the proliferation of Hep3B cells.

**Example C Xenograft tumor model**

BALB/c nu/nu female mice were inoculated with $5\times10^6$ human liver cancer cells Hep3B subcutaneously in the right anterior scapula, where a volume ratio of a cell suspension to matrigel was 1: 1 (0.2/mL/mouse). When an average tumor volume was 158 $mm^3$, the mice were randomly grouped according to a tumor size. The treatment group was administered with a solution of a to-be-tested compound prepared with an appropriate solvent, and the solvent control group was administered with a blank solvent. During treatment, the tumor volume was measured twice a week, and the tumor weight was determined after a last dose to determine the activity of the compound. A tumor growth inhibition (TGI) rate (%) was calculated by comparing the tumor volumes and weights of the treatment group and the solvent control group. Body weight measurement, as the routine determination of toxicity, has the same frequency as tumor volume measurement. In this model, the compound represented by Formula I of the present disclosure showed good anti-tumor activity. For example, when the dose was 50 mg/kg, 100 mg/kg and 200 mg/kg (BID× 14), the inhibition rate of the compound represented by Formula I on the tumor volume growth of Hep3B was 73.02%, 86.26% and 90.26%, respectively and the inhibition rate of the compound represented by Formula I on the tumor weight growth of HepB was 84.76%, 92.27% and 98.15%, respectively, indicating that the compound represented by Formula I showed a dose-dependent effect in inhibiting tumor volume and weight. In addition, during the entire assay, none of the animals administered with the compound represented by Formula I showed an obvious weight loss, indicating that the two compounds are well tolerated under the conditions of treatment doses.

**Claims**

1. A salt form of a compound represented by Formula I, or crystal forms thereof:

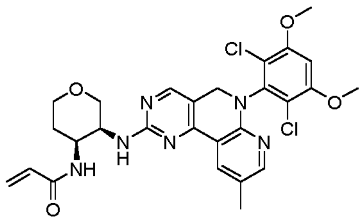

Formula I.

2. A salt form of a compound represented by Formula I, wherein the salt form is selected from a maleate, a phosphate, an L-tartrate or an adipate.

3. The salt form according to claim 2, wherein the salt form is a phosphate whose structure is represented by Formula II:

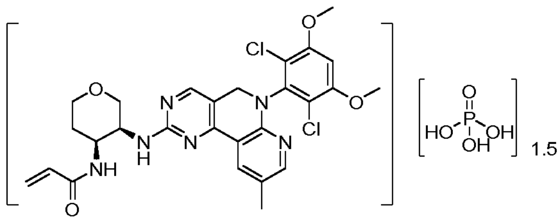

Formula II.

4. The salt form according to claim 3, wherein the phosphate is a phosphate crystal form 1, and an X-ray powder diffraction pattern of the phosphate crystal form 1 has characteristic peaks at diffraction angles 2θ of 4.9°±0.2°, 10.7°±0.2°, 16.8°±0.2° and 21.3°±0.2°.

5. The salt form according to claim 4, wherein the X-ray powder diffraction pattern of the phosphate crystal form 1 has characteristic peaks at diffraction angles 2θ of 4.9°±0.2°, 8.3°±0.2°, 10.7°±0.2°, 11.6°±0.2°, 12.8±0.2°, 16.8°±0.2° and 21.3°±0.2°.

6. The salt form according to claim 4 or 5, wherein the X-ray powder diffraction pattern of the phosphate crystal form 1 has characteristic peaks at diffraction angles 2θ of 4.9°±0.2°, 8.3°±0.2°, 10.7°±0.2°, 11.6°±0.2°, 12.8±0.2°, 16.8°±0.2°, 20.1°±0.2°, 21.3°±0.2° and 28.5°±0.2°.

7. The salt form according to any one of claims 4 to 6, wherein the phosphate crystal form 1 has an X-ray powder diffraction pattern substantially as shown in FIG. 9 or FIG. 10.

8. The salt form according to claim 2, wherein the salt form is a maleate.

9. The salt form according to claim 8, wherein the maleate is a maleate crystal form 1, and an X-ray powder diffraction pattern of the maleate crystal form 1 has characteristic peaks at diffraction angles 2θ of 3.7°±0.2°, 9.9°±0.2°, 16.2°±0.2° and 11.0°±0.2°.

10. The salt form according to claim 9, wherein the X-ray powder diffraction pattern of the maleate crystal form 1 has characteristic peaks at diffraction angles 2θ of 3.7°±0.2°, 9.9°±0.2°, 11.0°±0.2°, 13.4°±0.2°, 16.2±0.2°, 17.2°±0.2° and 20.6°±0.2°.

11. The salt form according to claim 8 or 9, wherein the X-ray powder diffraction pattern the maleate crystal form 1 has characteristic peaks at diffraction angles 2θ of 3.7°±0.2°, 9.9°±0.2°, 11.0°±0.2°, 13.4°±0.2°, 16.2°±0.2°,

17.2°±0.2°, 18.7°±0.2°, 19.4°±0.2°, 20.6°±0.2°, 22.6°±0.2° and 24.4°±0.2°.

**12.** The salt form according to any one of claims 9 to 11, wherein the maleate crystal form 1 has an X-ray powder diffraction pattern substantially as shown in FIG. 4.

**13.** The salt form or crystal form according to claim 1, wherein the crystal form is a crystal form A of the compound represented by Formula I, and an X-ray powder diffraction pattern of the crystal form A has characteristic peaks at diffraction angles 2θ7.5°±0.2°, 10.7°±0.2°, 11.3°±0.2° and 15.1°±0.2°.

**14.** The salt form or crystal form according to claim 13, wherein the X-ray powder diffraction pattern of the crystal form A has characteristic peaks at diffraction angles 2θ of 7.5°±0.2°, 10.7°±0.2°, 11.3°±0.2°, 13.1°±0.2°, 15.1°±0.2°, 20.9°±0.2° and 25.2°±0.2°.

**15.** The salt form or crystal form according to claim 13 or 14, wherein the X-ray powder diffraction pattern of the crystal form A has characteristic peaks at diffraction angles 2θ of 7.5°±0.2°, 10.7°±0.2°, 11.3°±0.2°, 13.1°±0.2°, 15.1°±0.2°, 20.9°±0.2°, 21.6°±0.2°, 22.8±0.2°, 23.5°±0.2°, 25.2°±0.2° and 30.2±0.2°.

**16.** The salt form or crystal form according to any one of claims 13 to 15, wherein the crystal form A has an X-ray powder diffraction pattern substantially as shown in FIG. 1.

**17.** The salt form according to claim 2, wherein the salt form is an L-tartrate, the L-tartrate is an L-tartrate crystal form 1, and an X-ray powder diffraction pattern of the L-tartrate crystal form 1 has characteristic peaks at diffraction angles 2θ of 5.4°±0.2°, 17.6°±0.2°, 19.7°±0.2° and 20.7°±0.2°.

**18.** The salt form according to claim 17, wherein the X-ray powder diffraction pattern of the L-tartrate crystal form 1 has characteristic peaks at diffraction angles 2θ of 5.4°±0.2°, 12.9°±0.2°, 16.5°±0.2°, 17.6°±0.2°, 19.7±0.2° and 20.7°±0.2°.

**19.** The salt form according to claim 17 or 18, wherein the X-ray powder diffraction pattern of the L-tartrate crystal form 1 has characteristic peaks at diffraction angles 2θ of 5.4°±0.2°, 12.9°±0.2°, 16.5°±0.2°, 17.6°±0.2°, 19.7±0.2°, 20.7°±0.2°, 22.2°±0.2° and 26.4°±0.2°.

**20.** The salt form according to any one of claims 17 to 19, wherein the L-tartrate crystal form 1 has an X-ray powder diffraction pattern substantially as shown in FIG. 13.

**21.** The salt form according to claim 2, wherein the salt form is an adipate, the adipate is an adipate crystal form 1, and an X-ray powder diffraction pattern of the adipate crystal form 1 has characteristic peaks at diffraction angles 2θ of 5.8°±0.2°, 8.4°±0.2°, 12.3°±0.2° and 22.9°±0.2°.

**22.** The salt form according to claim 21, wherein the X-ray powder diffraction pattern of the adipate crystal form 1 has characteristic peaks at diffraction angles 2θ of 5.8°±0.2°, 8.4°±0.2°, 10.0°±0.2°, 10.4°±0.2°, 12.3±0.2°, 17.5°±0.2° and 22.9°±0.2°.

**23.** The salt form according to claim 21 or 22, wherein the X-ray powder diffraction pattern of the adipate crystal form 1 has characteristic peaks at diffraction angles 2θ of 5.8°±0.2°, 8.4°±0.2°, 10.0°±0.2°, 10.4°±0.2°, 12.3±0.2°, 17.5°±0.2°, 22.9°±0.2°, 25.4°±0.2° and 25.9°±0.2°.

**24.** The salt form according to any one of claims 21 to 23, wherein the adipate crystal form 1 has an X-ray powder diffraction pattern substantially as shown in FIG. 16.

**25.** A pharmaceutical composition, comprising a therapeutically effective amount of the salt form or crystal form according to any one of claims 1 to 24 and a pharmaceutically acceptable excipient, adjuvant and/or carrier.

**26.** A use of the salt form or crystal form according to any one of claims 1 to 24 or the composition according to claim 25 for preparing a medicament for treatment, prophylaxis, and delaying or preventing the occurrence or progression of cancer or cancer metastasis.

**27.** A use of the salt form or crystal form according to any one of claims 1 to 24 or the composition according to claim

25 for preparing a medicament for treating an FGFR4-mediated disease.

**28.** The use according to claim 27, wherein the FGFR4-mediated disease is cancer.

**29.** The use according to claim 26 or 28, wherein the cancer is selected from breast cancer, multiple myeloma, bladder cancer, endometrial cancer, gastric cancer, cervical cancer, rhabdomyosarcoma, non-small-cell lung carcinoma, small-cell lung carcinoma, pleomorphic lung cancer, ovarian cancer, esophageal carcinoma, melanoma, colorectal carcinoma, hepatocellular carcinoma, head and neck neoplasm, intracranial tumor, hepatocholangiocarcinoma, myelodysplastic syndrome, glioblastoma, prostate cancer, thyroid cancer, Schwann cell tumor, squamous-cell lung carcinoma, lichenoid keratosis, synoviosarcoma, skin cancer, pancreatic cancer, testicular cancer or liposarcoma.

**30.** A method for treating cancer, comprising administering a therapeutically effective amount of the salt form or crystal form according to any one of claims 1 to 24 or the composition according to claim 25 to a subject in need, wherein the cancer is breast cancer, multiple myeloma, bladder cancer, endometrial cancer, gastric cancer, cervical cancer, rhabdomyosarcoma, non-small-cell lung carcinoma, small-cell lung carcinoma, pleomorphic lung cancer, ovarian cancer, esophageal carcinoma, melanoma, colorectal carcinoma, hepatocellular carcinoma, head and neck neoplasm, hepatocholangiocarcinoma, myelodysplastic syndrome, glioblastoma, prostate cancer, thyroid cancer, Schwann cell tumor, squamous-cell lung carcinoma, lichenoid keratosis, synoviosarcoma, skin cancer, pancreatic cancer, testicular cancer or liposarcoma.

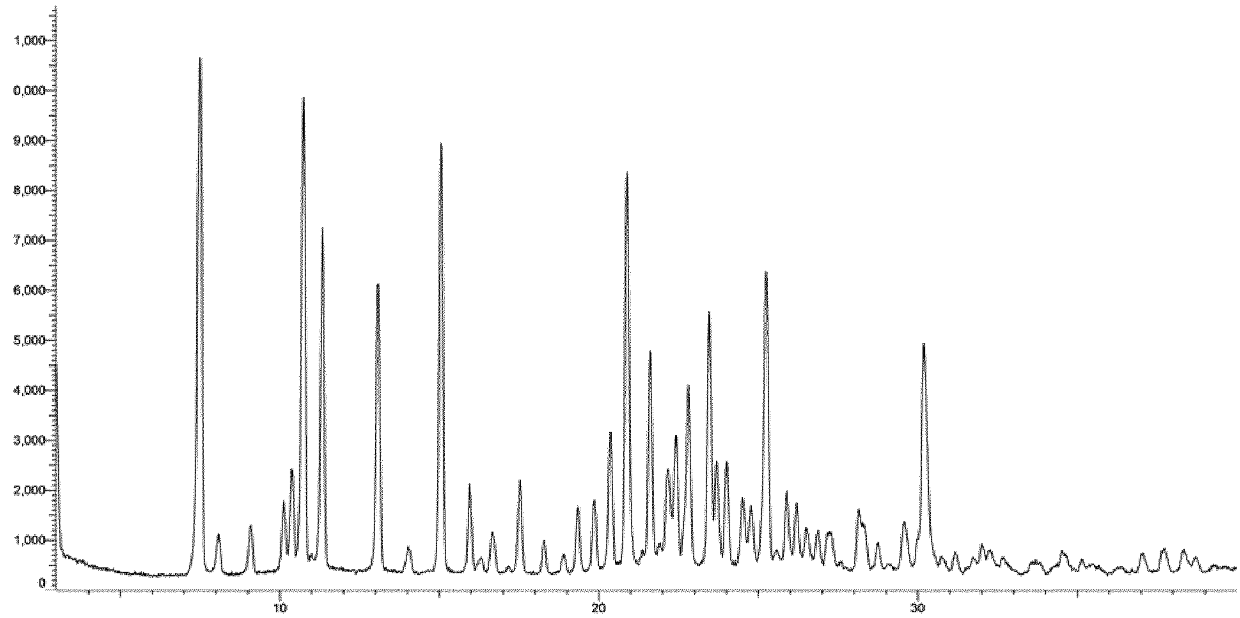
1,000
0,000
9,000
8,000
7,000
6,000
5,000
4,000
3,000
2,000
1,000
0
10
20
30

FIG. 1

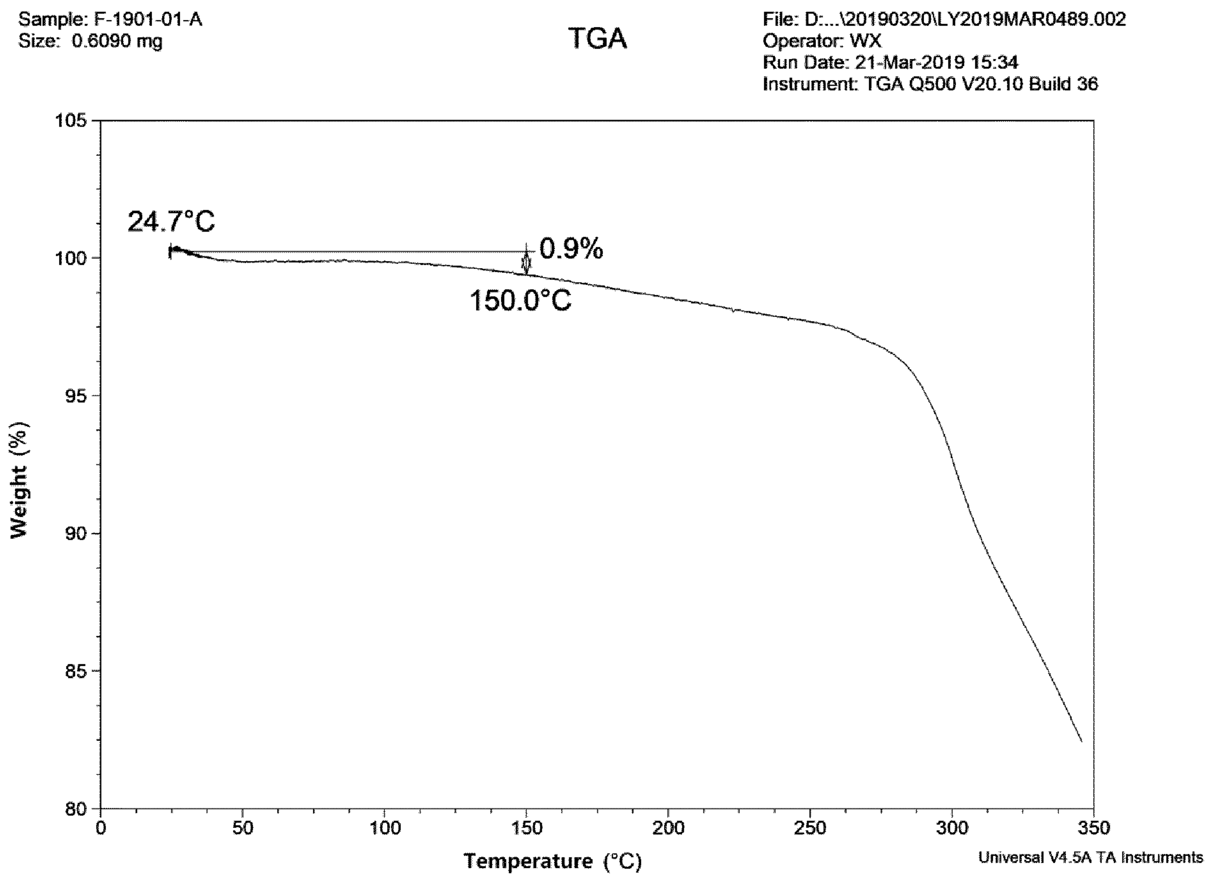
Sample: F-1901-01-A
Size: 0.6090 mg
TGA
File: D:...\20190320\LY2019MAR0489.002
Operator: WX
Run Date: 21-Mar-2019 15:34
Instrument: TGA Q500 V20.10 Build 36
24.7°C
0.9%
150.0°C
Weight (%)
105
100
95
90
85
80
0
50
100
150
200
250
300
350
Temperature (°C)
Universal V4.5A TA Instruments

FIG. 2

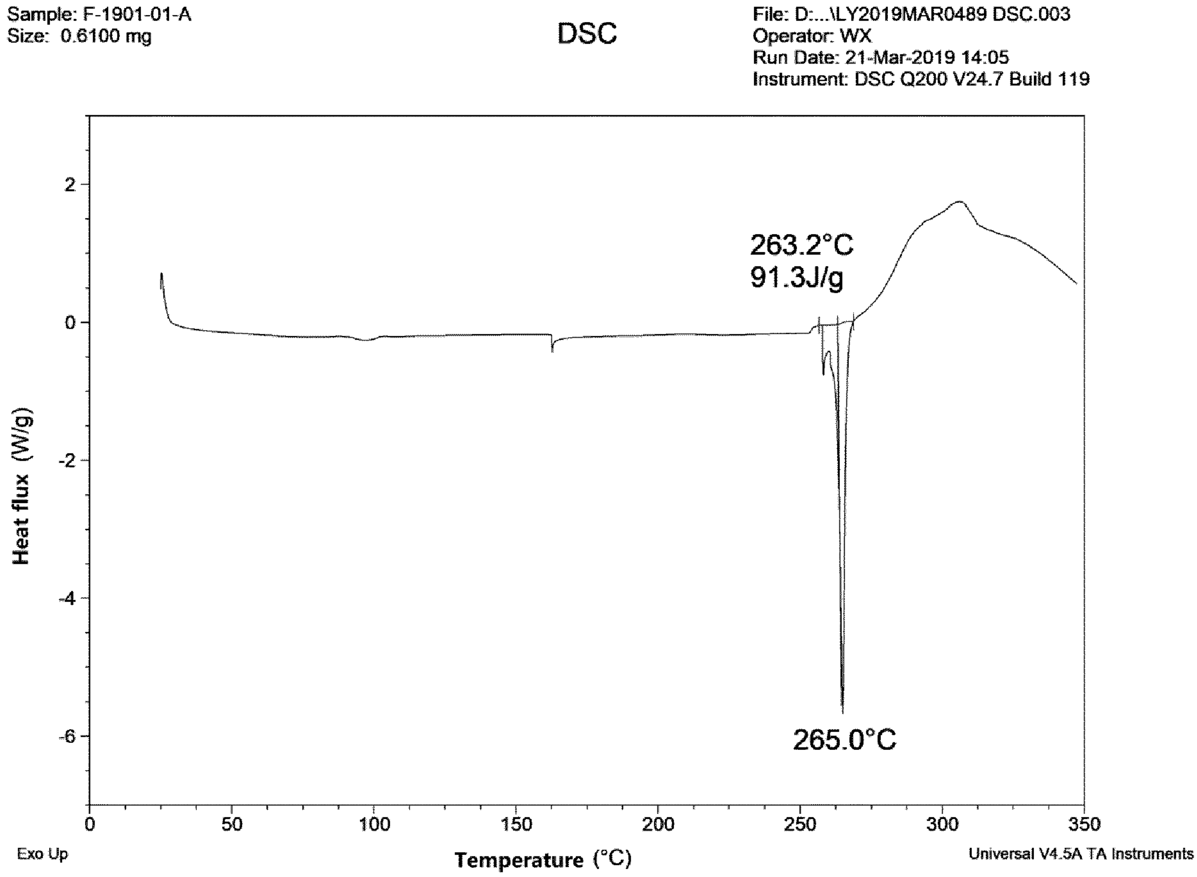
Sample: F-1901-01-A
Size: 0.6100 mg
DSC
File: D:...\LY2019MAR0489 DSC.003
Operator: WX
Run Date: 21-Mar-2019 14:05
Instrument: DSC Q200 V24.7 Build 119
263.2°C
91.3J/g
265.0°C
Heat flux (W/g)
Temperature (°C)
Exo Up
Universal V4.5A TA Instruments
2
0
-2
-4
-6
0
50
100
150
200
250
300
350

FIG. 3

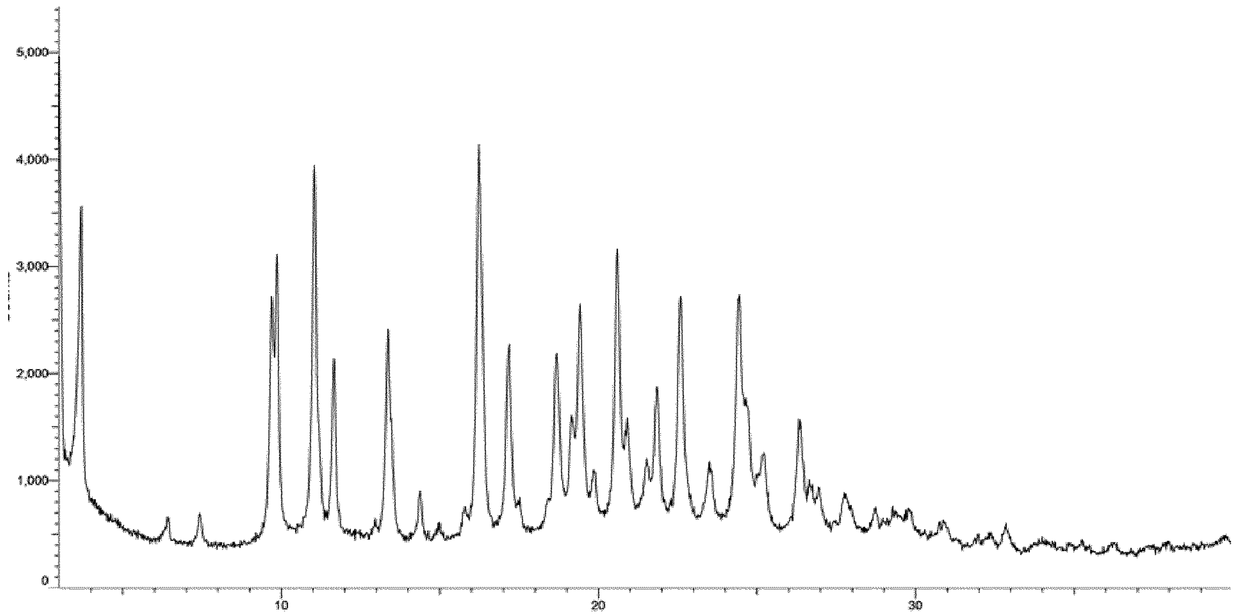
5,000
4,000
3,000
2,000
1,000
0
10
20
30

FIG. 4

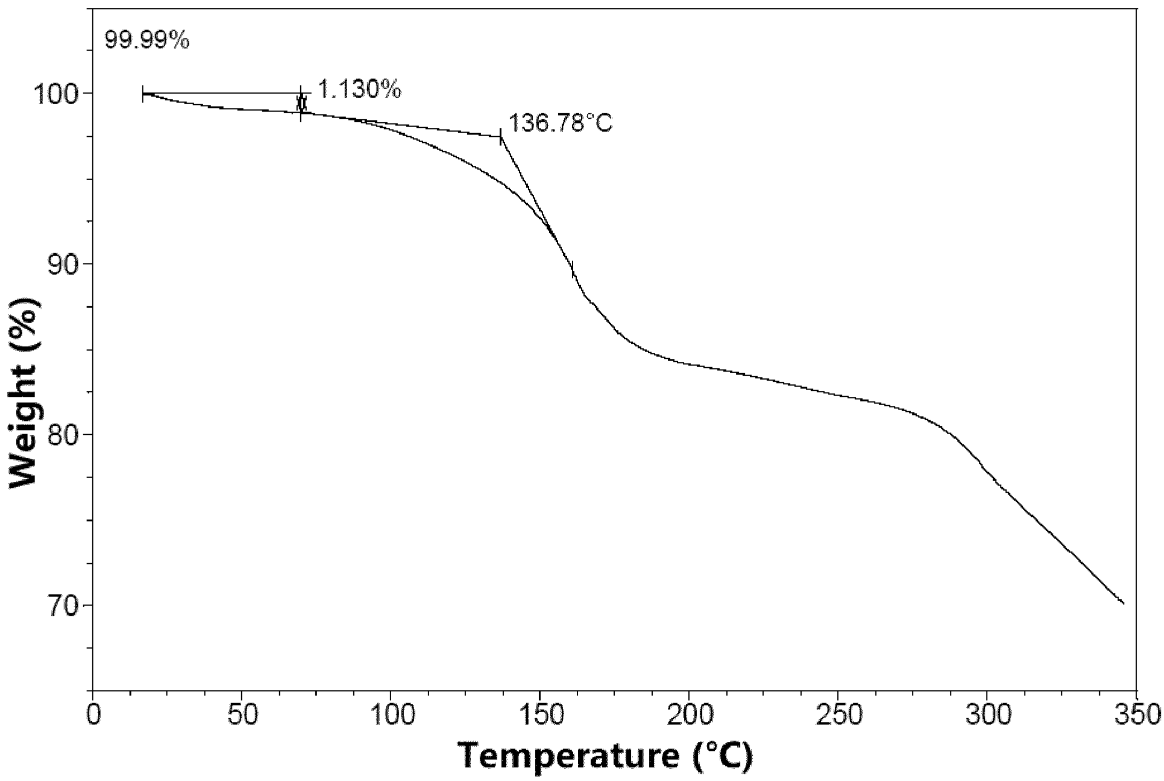
99.99%
1.130%
136.78°C
100
90
80
70
Weight (%)
0
50
100
150
200
250
300
350
Temperature (°C)

FIG. 5

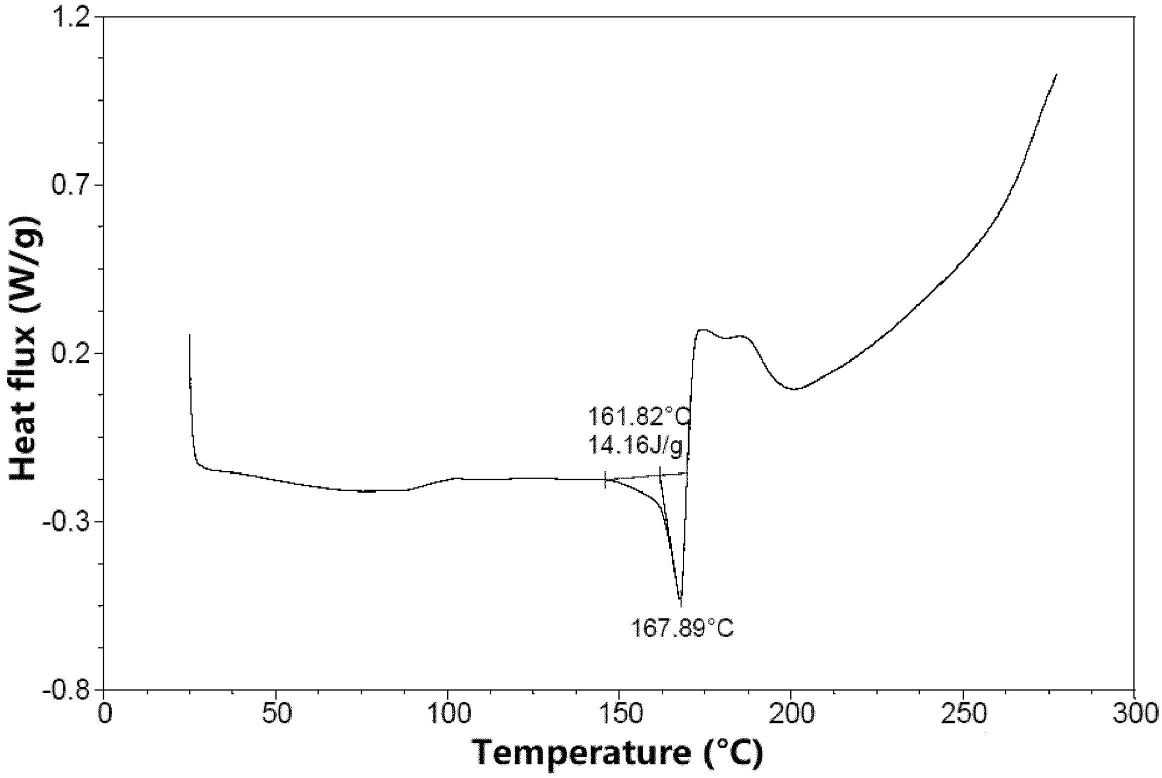
1.2
0.7
0.2
-0.3
-0.8
Heat flux (W/g)
161.82°C
14.16J/g
167.89°C
0
50
100
150
200
250
300
Temperature (°C)

FIG. 6

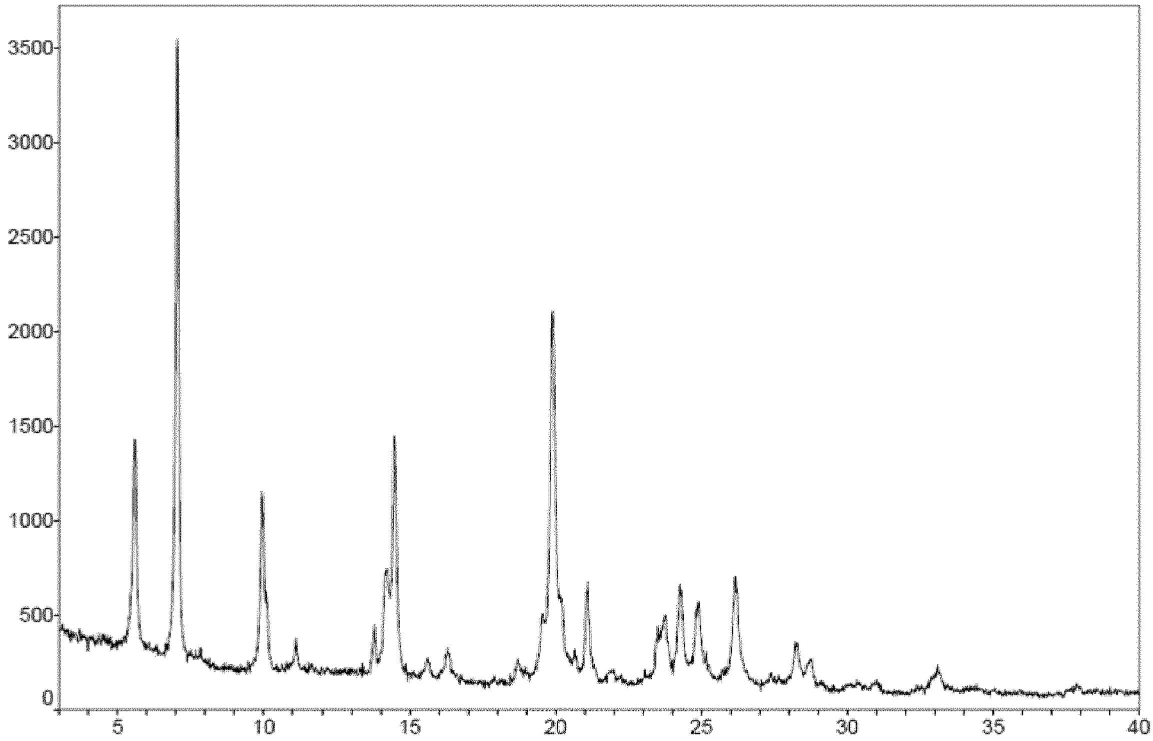
3500
3000
2500
2000
1500
1000
500
0
5
10
15
20
25
30
35
40

FIG. 7

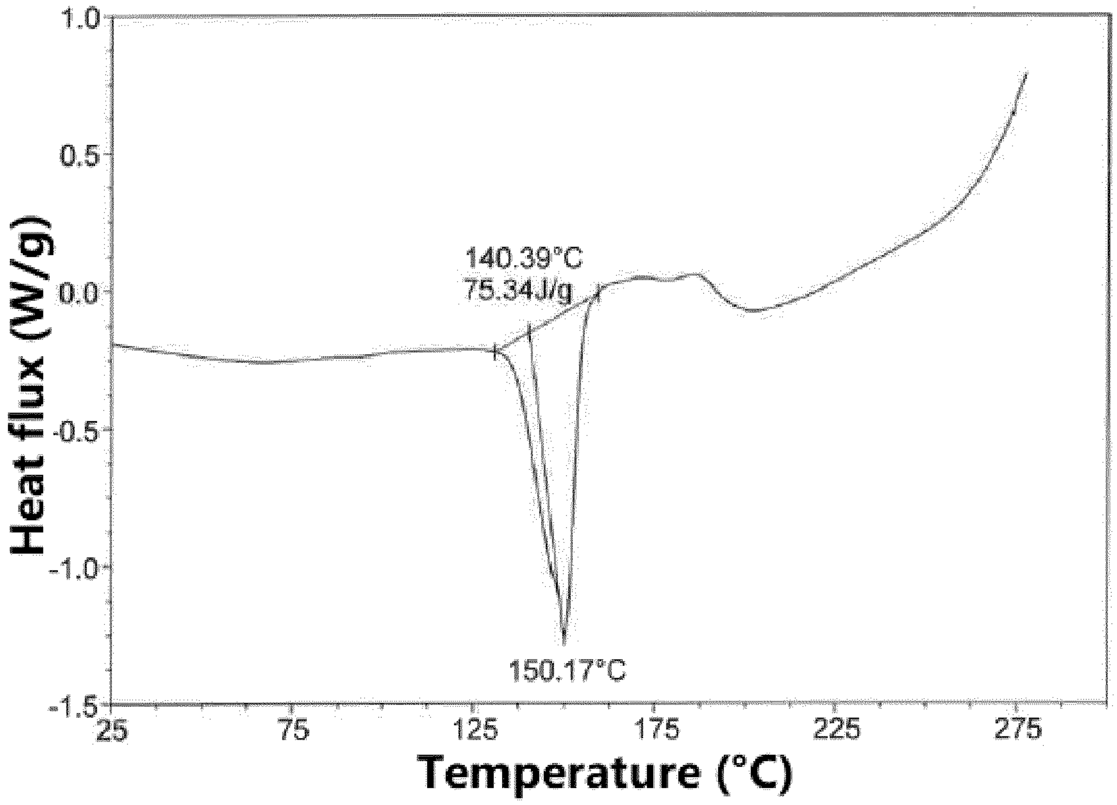
1.0
0.5
0.0
-0.5
-1.0
-1.5
Heat flux (W/g)
140.39°C
75.34J/g
150.17°C
25
75
125
175
225
275
Temperature (°C)

FIG. 8

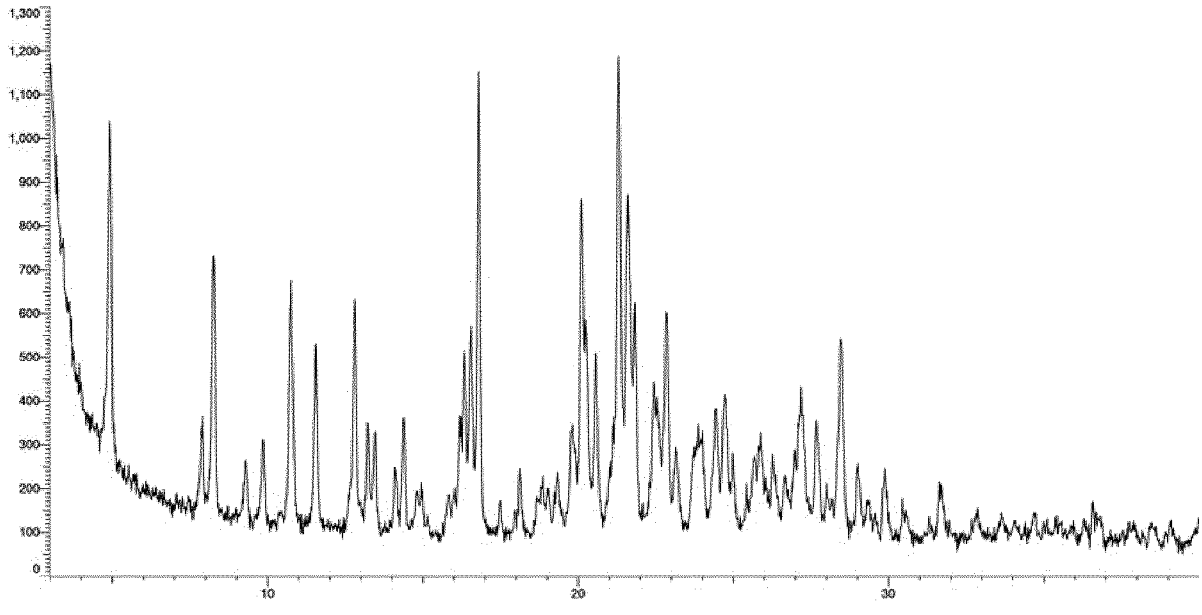
1,300
1,200
1,100
1,000
900
800
700
600
500
400
300
200
100
0
10
20
30

FIG. 9

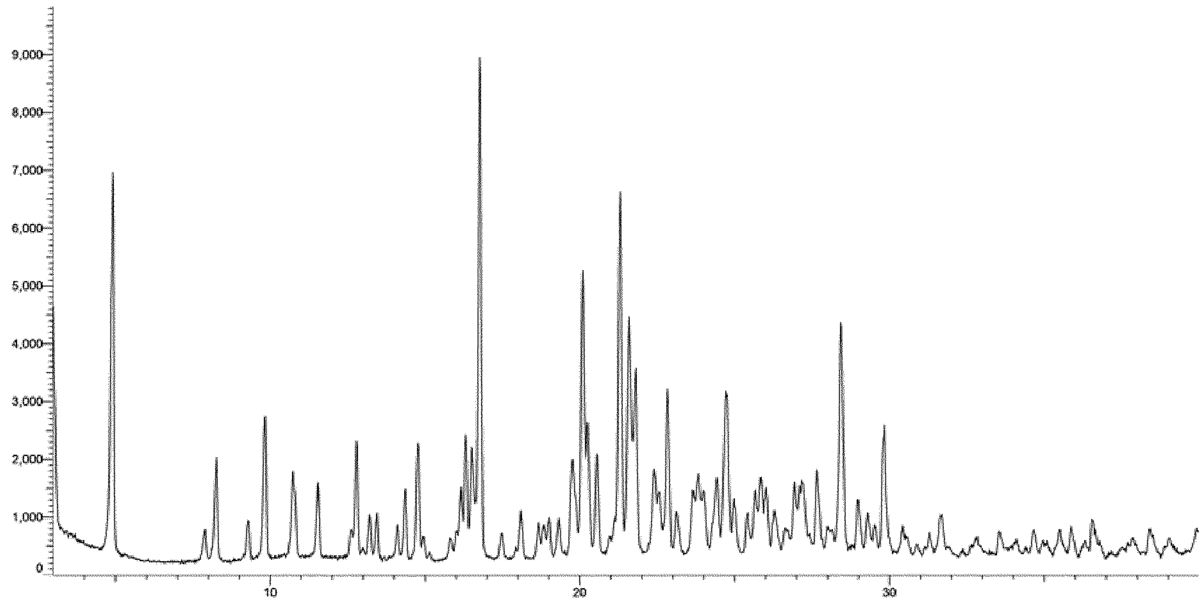
9,000
8,000
7,000
6,000
5,000
4,000
3,000
2,000
1,000
0
10
20
30

FIG. 10

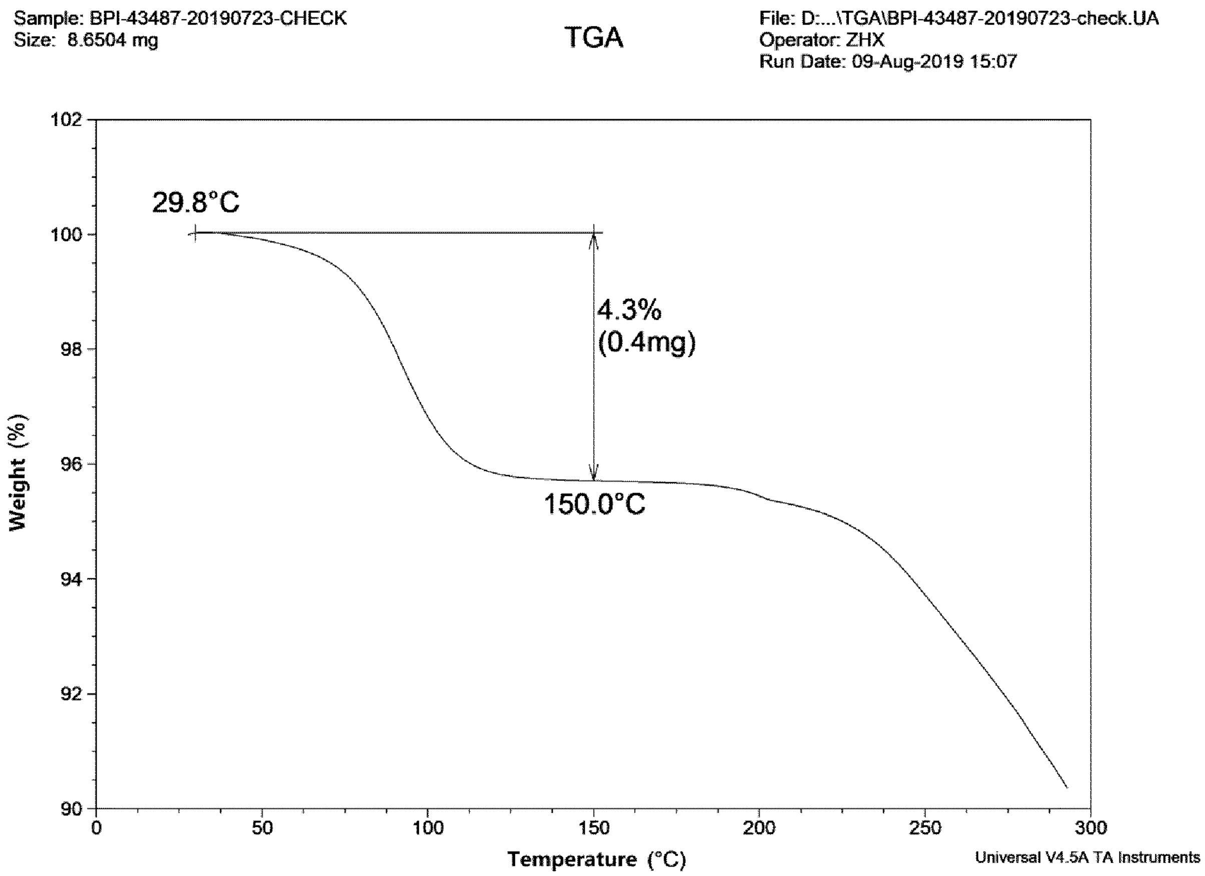
Sample: BPI-43487-20190723-CHECK
Size: 8.6504 mg
TGA
File: D:...\TGA\BPI-43487-20190723-check.UA
Operator: ZHX
Run Date: 09-Aug-2019 15:07
29.8°C
4.3%
(0.4mg)
150.0°C
Weight (%)
Temperature (°C)
Universal V4.5A TA Instruments

FIG. 11

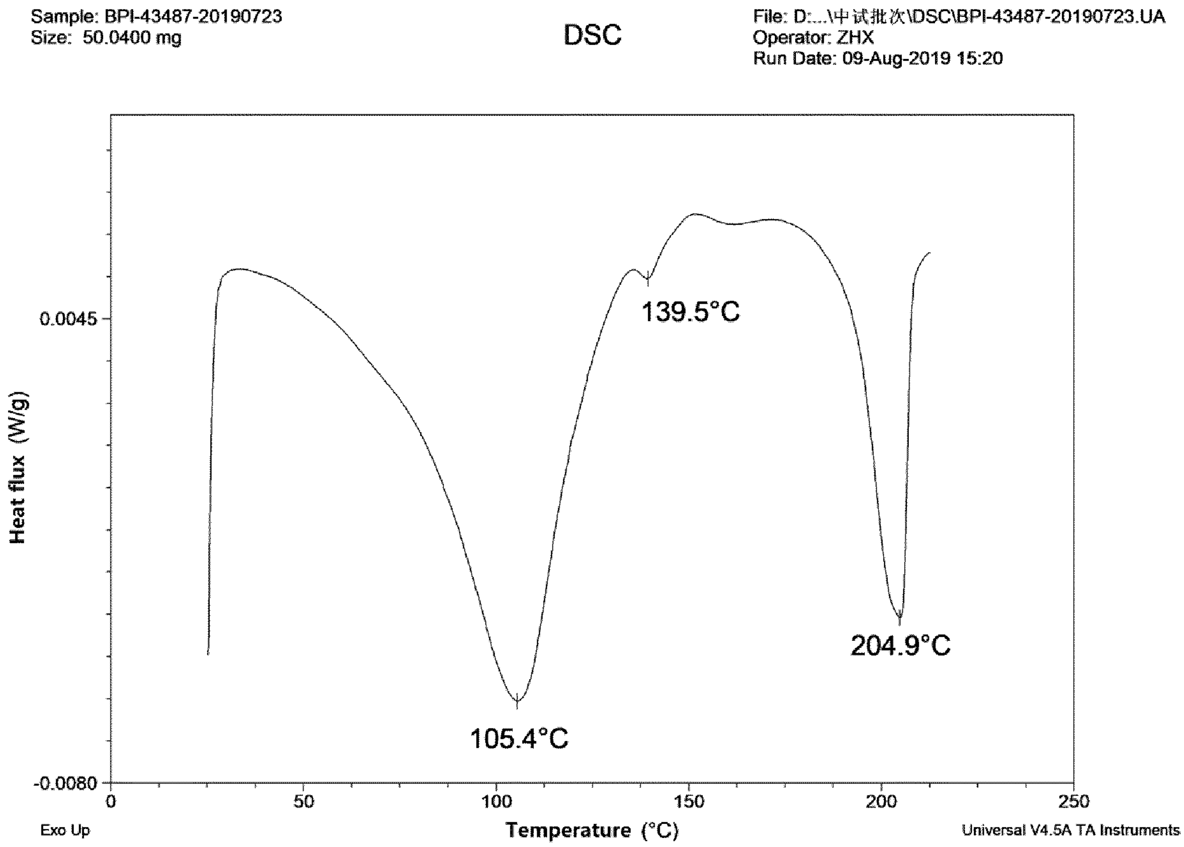
Sample: BPI-43487-20190723
Size: 50.0400 mg
DSC
File: D:...\中试批次\DSC\BPI-43487-20190723.UA
Operator: ZHX
Run Date: 09-Aug-2019 15:20
0.0045
-0.0080
Heat flux (W/g)
139.5°C
204.9°C
105.4°C
0
50
100
150
200
250
Exo Up
Temperature (°C)
Universal V4.5A TA Instruments

FIG. 12

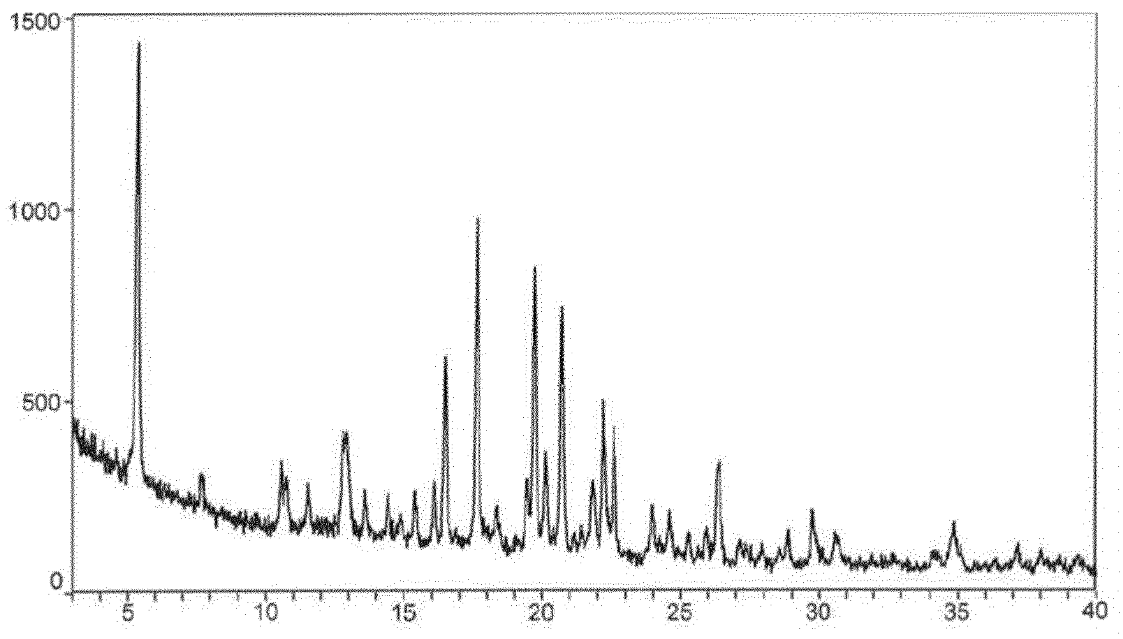
1500
1000
500
0
5
10
15
20
25
30
35
40

FIG. 13

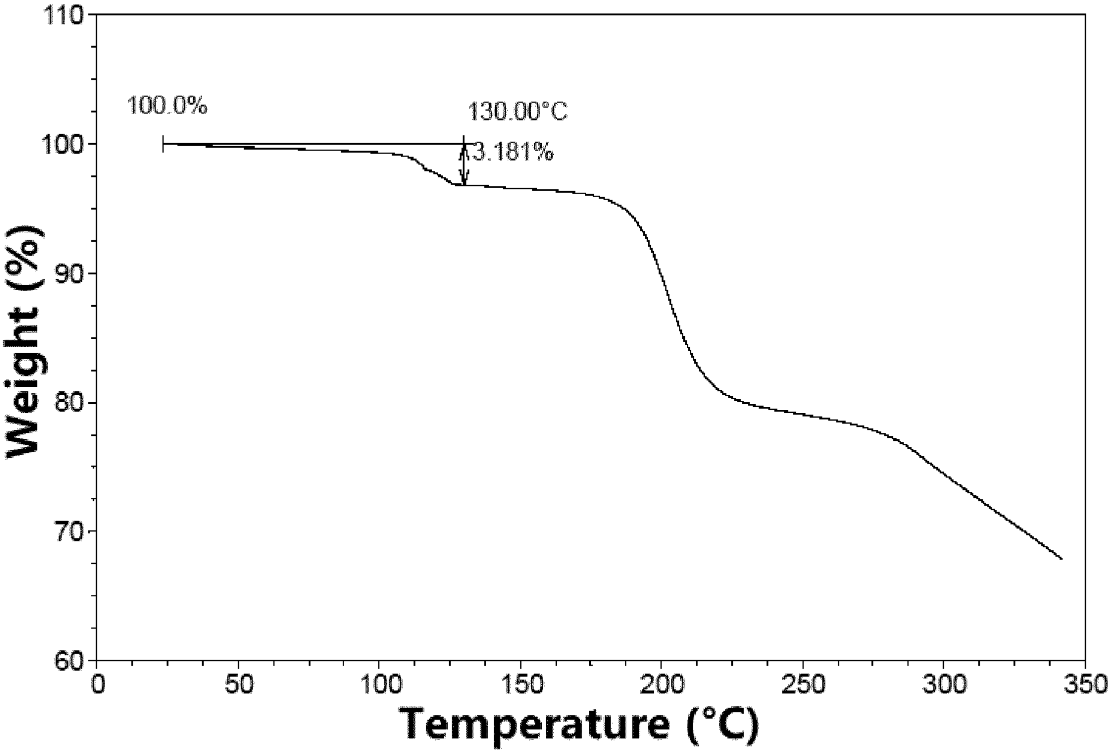
100.0%
130.00°C
3.181%
Weight (%)
Temperature (°C)
110
100
90
80
70
60
0
50
100
150
200
250
300
350

FIG. 14

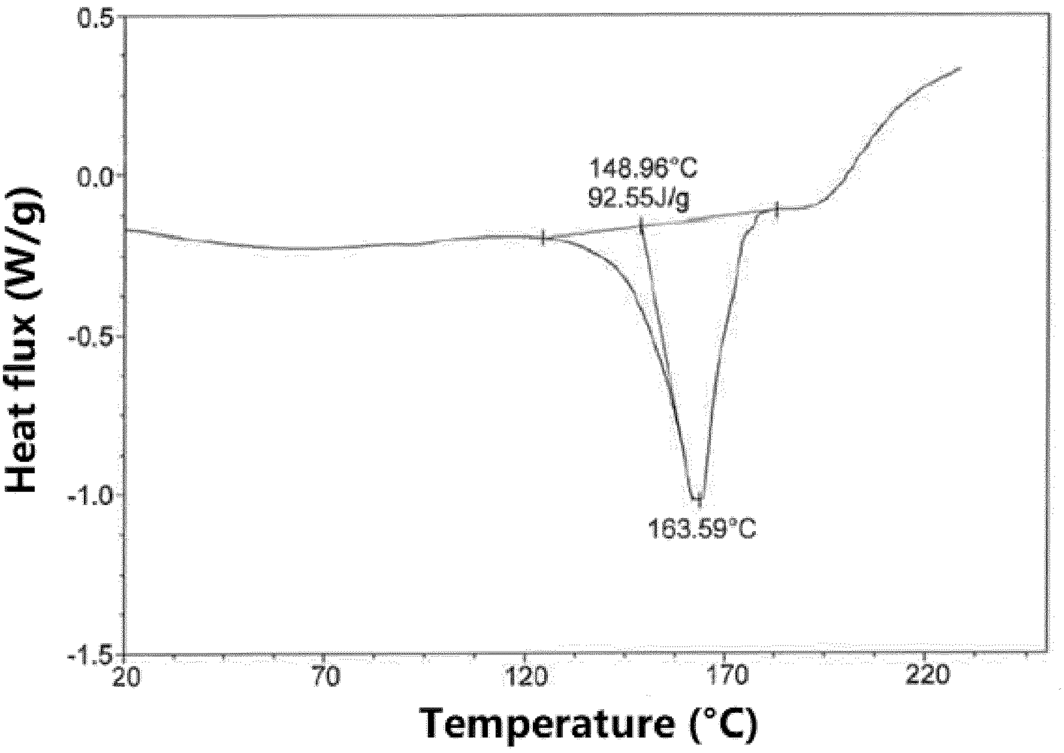
148.96°C
92.55J/g
163.59°C
Heat flux (W/g)
Temperature (°C)
0.5
0.0
-0.5
-1.0
-1.5
20
70
120
170
220

FIG. 15

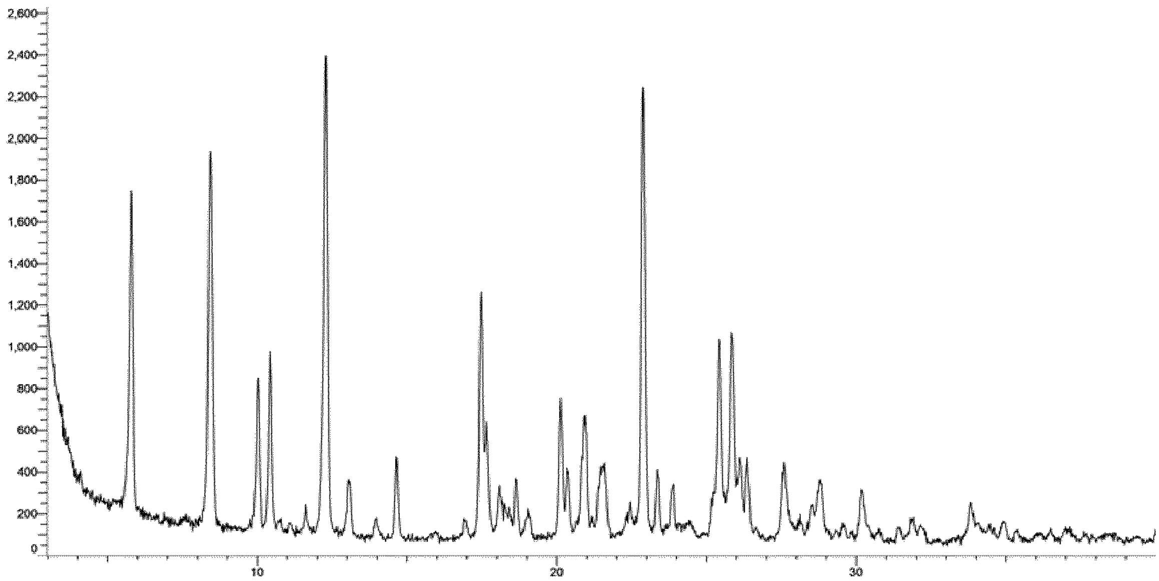
2,600
2,400
2,200
2,000
1,800
1,600
1,400
1,200
1,000
800
600
400
200
0
10
20
30

FIG. 16

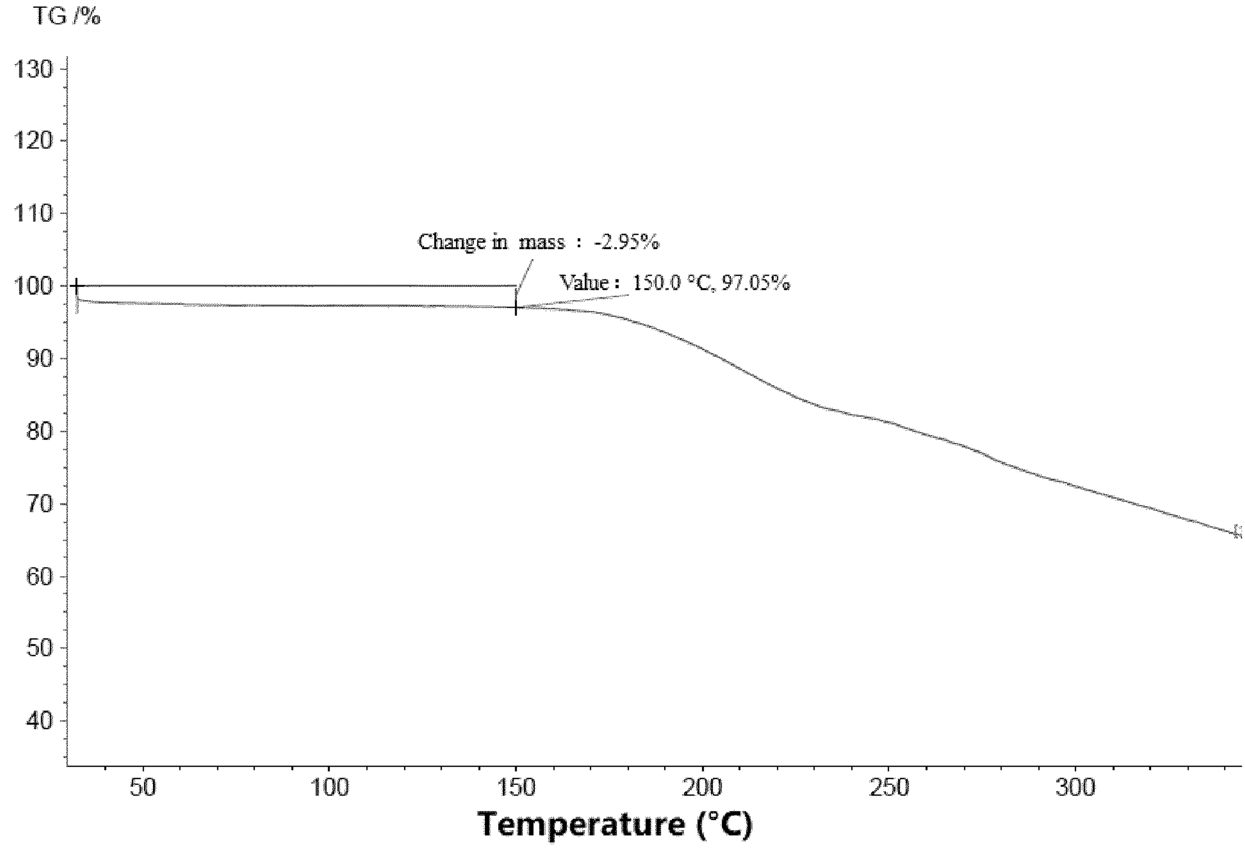
TG /%
130
120
110
100
90
80
70
60
50
40
Change in mass : -2.95%
Value : 150.0 °C, 97.05%
50
100
150
200
250
300
Temperature (°C)

FIG. 17

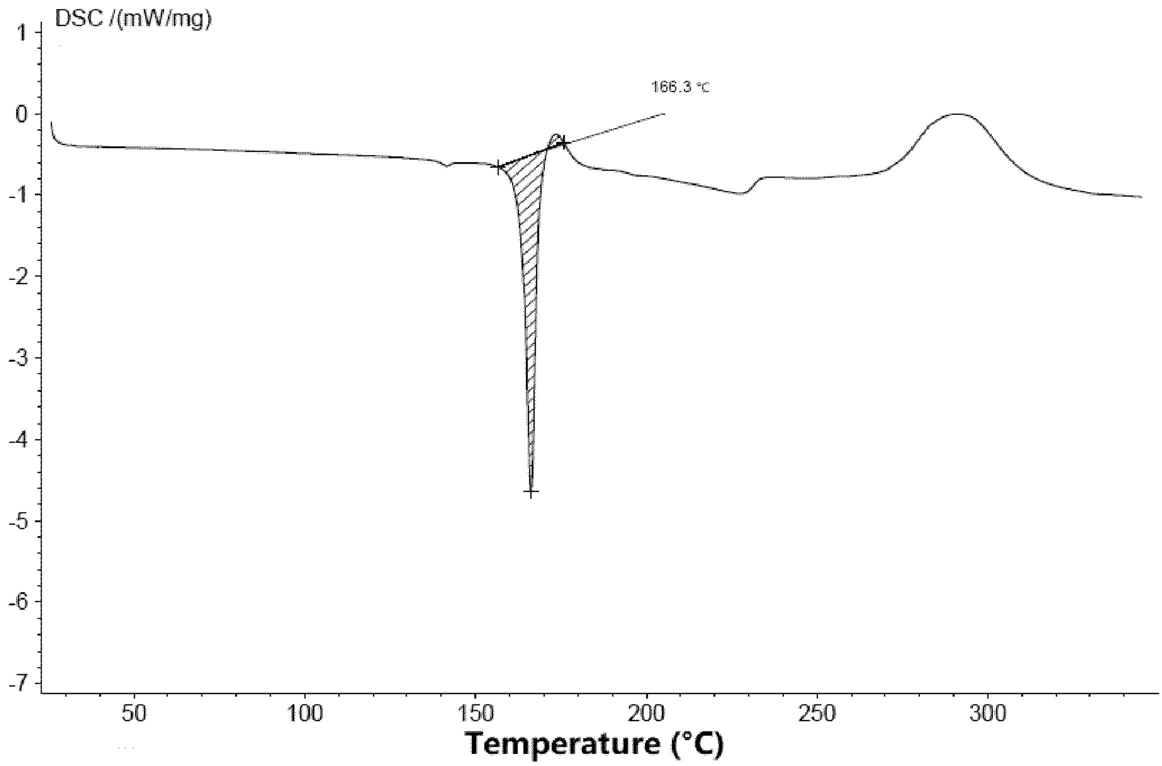
DSC /(mW/mg)
166.3 °C
1
0
-1
-2
-3
-4
-5
-6
-7
50
100
150
200
250
300
Temperature (°C)

FIG. 18

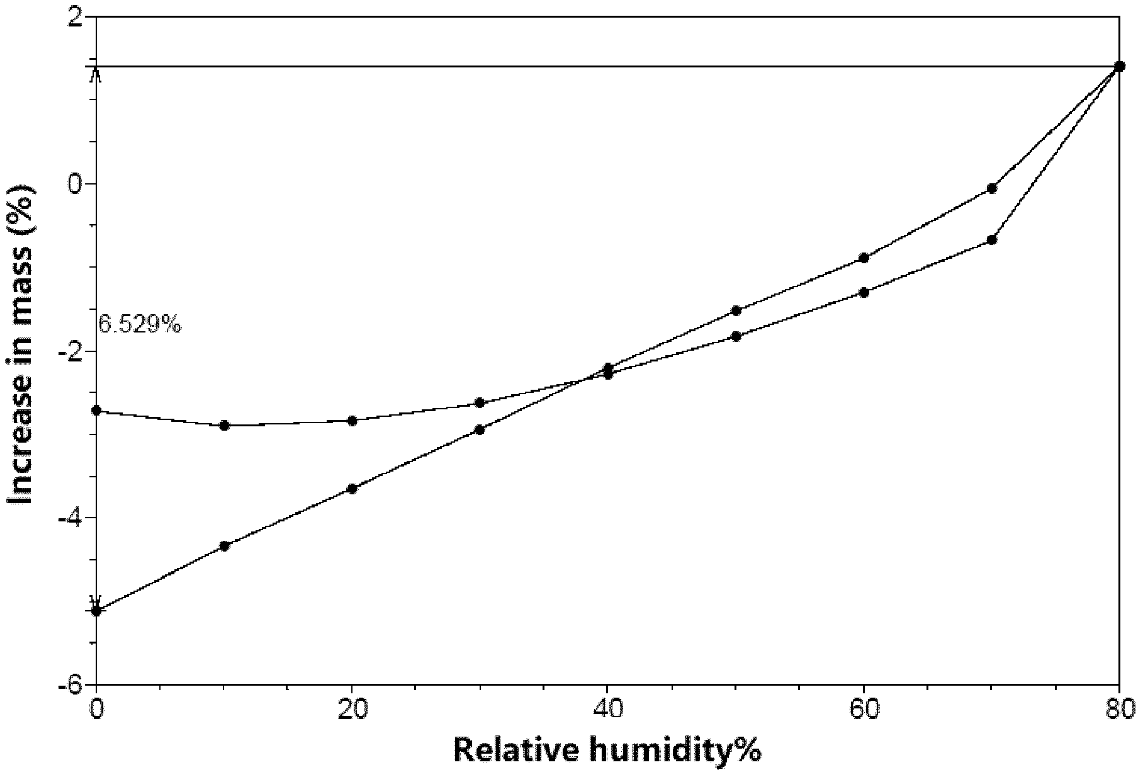
Increase in mass (%)
6.529%
2
0
-2
-4
-6
0
20
40
60
80
Relative humidity%

FIG. 19

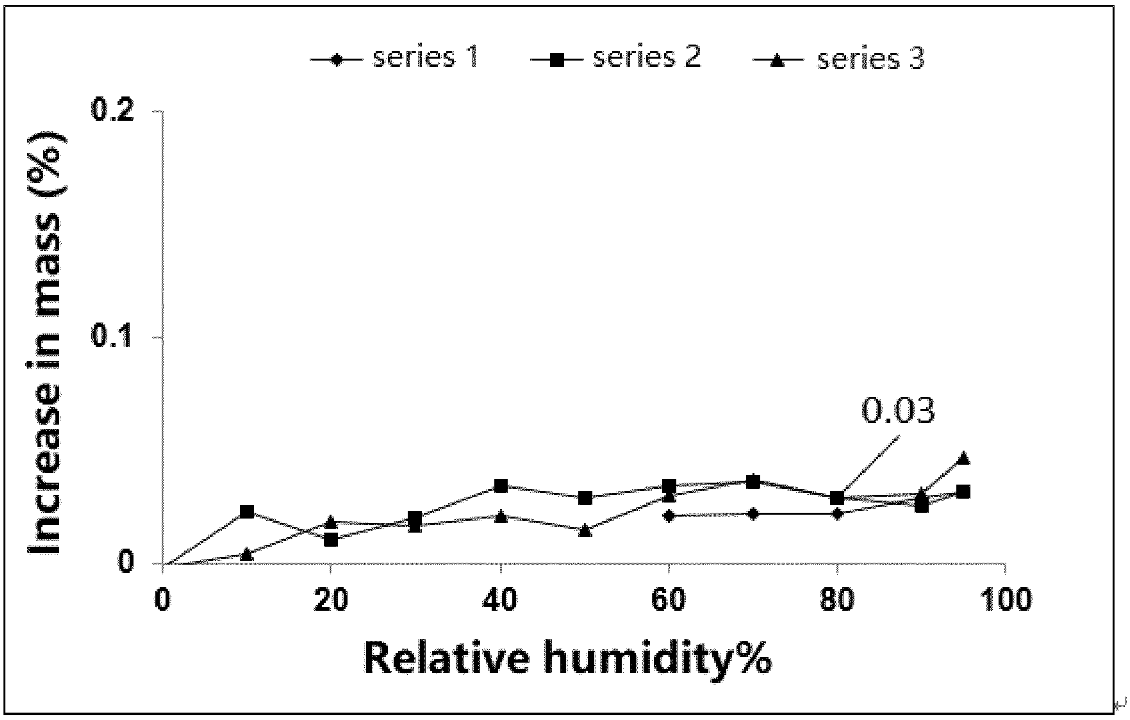
series 1
series 2
series 3
Increase in mass (%)
0.2
0.1
0
0.03
0
20
40
60
80
100
Relative humidity%

FIG. 20

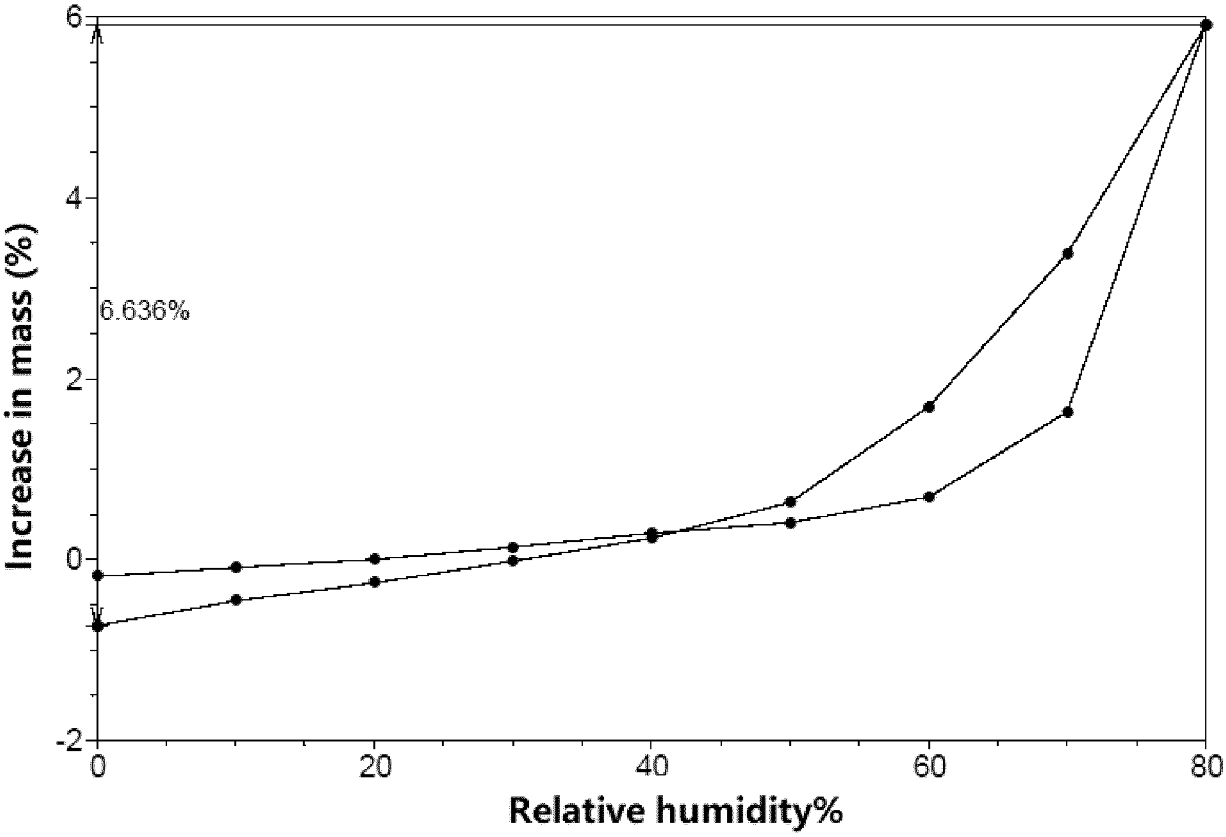
Increase in mass (%)
6
4
2
0
-2
6.636%
0
20
40
60
80
Relative humidity%

FIG. 21

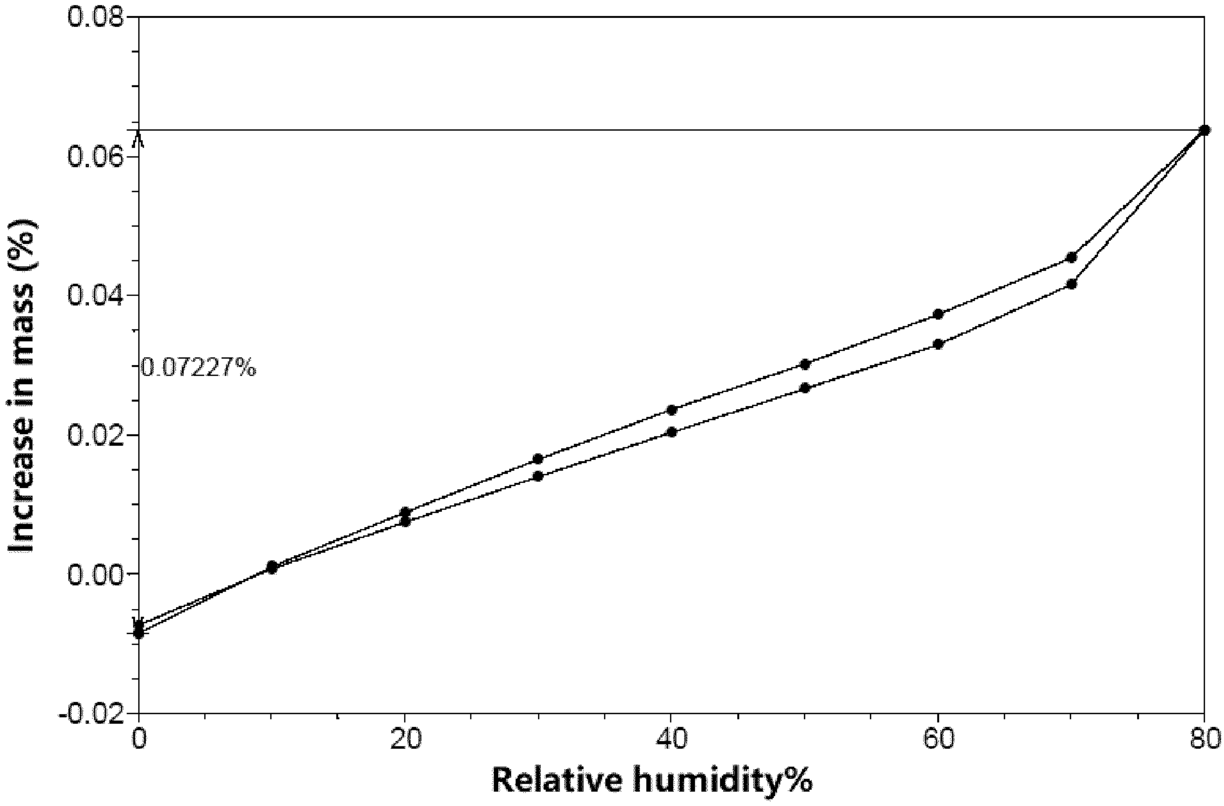
0.08
0.06
0.04
0.02
0.00
-0.02
0.07227%
Increase in mass (%)
0
20
40
60
80
Relative humidity%

FIG. 22

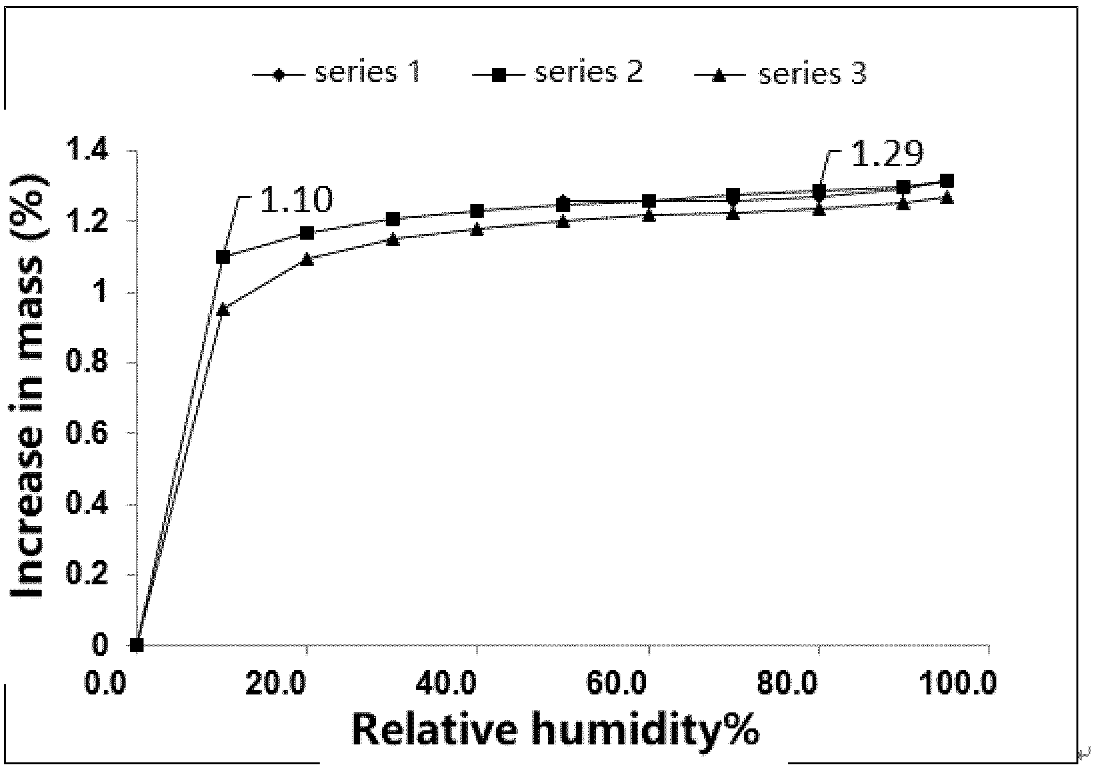
series 1
series 2
series 3
1.10
1.29
1.4
1.2
1
0.8
0.6
0.4
0.2
0
Increase in mass (%)
0.0
20.0
40.0
60.0
80.0
100.0
Relative humidity%

FIG. 23

## INTERNATIONAL SEARCH REPORT

International application No.
**PCT/CN2021/083204**

**A. CLASSIFICATION OF SUBJECT MATTER**

C07D 487/14(2006.01)i； A61K 31/4375(2006.01)i； A61K 31/437(2006.01)i； A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

VEN, CNABS, CNKI, CA, STN: 贝达药业, FGFR4, 肿瘤, 癌, 吡喃, 二氢嘧啶并萘啶, fibroblast growth factor receptor, tumor, cancer, 酪氨酸蛋白激酶, protein tyrosine kinase

**C. DOCUMENTS CONSIDERED TO BE RELEVANT**

| Category* | Citation of document, with indication, where appropriate, of the relevant passages | Relevant to claim No. |
|---|---|---|
| PX | WO 2020063788 A1 (BETTA PHARMACEUTICALS CO., LTD.) 02 April 2020 (2020-04-02)<br>description, page 25, compound 13 | 1-29 |
| A | WO 2018153373 A1 (BETTA PHARMACEUTICALS CO., LTD.) 30 August 2018 (2018-08-30)<br>embodiment 2 | 1-29 |
| A | CN 102216300 A (ZHEJIANG BETA PHARMA INC.) 12 October 2011 (2011-10-12)<br>entire document | 1-29 |
| A | CN 110022900 A (BLUEPRINT MEDICINES CORPORATION) 16 July 2019 (2019-07-16)<br>entire document | 1-29 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 June 2021** | **12 July 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No. **PCT/CN2021/083204**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **30**
because they relate to subject matter not required to be searched by this Authority, namely:

[1] Claim 30 relates to a method of treatment of the human or animal body by therapy and therefore the subject matter of claim 30 does not warrant an international search according to the criteria set out in PCT Rule 39.1(iv). (PCT Rule 39.1(iv).)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/083204**

| Patent document cited in search report | | | Publication date (day/month/year) | Patent family member(s) | | | Publication date (day/month/year) |
|---|---|---|---|---|---|---|---|
| WO | 2020063788 | A1 | 02 April 2020 | TW | 202028211 | A | 01 August 2020 |
| WO | 2018153373 | A1 | 30 August 2018 | TW | 201831483 | A | 01 September 2018 |
| | | | | JP | 2020509089 | A | 26 March 2020 |
| | | | | EP | 3587419 | A1 | 01 January 2020 |
| | | | | KR | 20190126344 | A | 11 November 2019 |
| | | | | AU | 2018226315 | B2 | 28 January 2021 |
| | | | | EP | 3587419 | A4 | 05 August 2020 |
| | | | | SG | 11201907909T | A | 27 September 2019 |
| | | | | AU | 2018226315 | A1 | 03 October 2019 |
| | | | | RU | 2745035 | C1 | 18 March 2021 |
| | | | | CA | 3054455 | A1 | 30 August 2018 |
| | | | | CN | 110382499 | A | 25 October 2019 |
| CN | 102216300 | A | 12 October 2011 | CN | 102216300 | B | 22 October 2014 |
| | | | | WO | 2011038579 | A1 | 07 April 2011 |
| CN | 110022900 | A | 16 July 2019 | WO | 2018049233 | A9 | 12 July 2018 |
| | | | | US | 2019192522 | A1 | 27 June 2019 |
| | | | | WO | 2018049233 | A1 | 15 March 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015061572 A


### Non-patent literature cited in the description


- **JOUANNEAU J et al.** *Oncogene,* 1999, vol. 18, 327-333
- **KOSTRZEWA M.** *Mammalian genome,* 1998, vol. 9 (2), 131-135
- **ESWARAKUMAR V P et al.** *Cytokine Growth Factor Rev,* 2005, vol. 16 (2), 139-149
- **SCHLESSINGER J et al.** *Mol Cell,* 2000, vol. 6, 743-750
- **DAILEY L et al.** *Cytokine Growth Factor Rev,* 2005, vol. 16, 233-247